# FASCICULE DE BREVET EUROPEEN

(11) **EP 0 566 446 B1**
(45) Date de publication et mention de la délivrance du brevet: **07.02.1996**
(21) Numéro de dépôt: 93400841.8
(22) Date de dépôt: 01.04.1993
(51) Int. Cl.: C07C 281/12, C07C 281/06, C07C 281/02, C07D 213/87, C07D 213/86, C07D 333/38, C07C 337/06, C07C 337/08, C07C 233/32, A61K 31/175, C07D 213/88

(54) **Dérivés d'indane-1, 3-dione et d'indane-1,2,3-trione, leurs procédés de préparation et leur application en thérapeutique**
Indan-1,3-Dion und Indan-1,2,3-Trionderivate, Verfahren zu ihrer Herstellung und ihrer therapeutische Verwendung
Derivatives of indane-1,3-dione and indane-1,2,3-trione, processes for their preparation and their application in therapy

(30) Priorité: 03.04.1992 FR 9204071
(43) Date de publication de la demande: 20.10.1993
(73) Titulaire: INNOTHERA, F-94110 Arcueil (FR)
(72) Inventeur: Cugnon de Sevricourt, Michel, F-14370 Moult (FR); Dacquet, Catherine, F-75005 Paris (FR); Finet, Michel, F-94260 Fresnes (FR); Le Marquer, Florence, F-75005 Paris (FR); Robba, Max, F-75004 Paris (FR); Tembo, Norbert Olivier, F-95800 Cergy Saint Christophe (FR); Yannic-Arnoult, Sylvie, F-91350 Grigny (FR); Torregrosa, Jean-Luc, F-93200 Saint Denis (FR)
(74) Mandataire: Kedinger, Jean-Paul

(56) Documents cités:
- EP-A- 0 172 128
- EP-A- 0 236 151
- EP-A- 0 456 133
- DE-A- 2 127 982
- US-A- 3 280 185
- JOURNAL OF ORGANIC CHEMISTRY. vol. 38, no. 12, 15 Juin 1973, EASTON US pages 2251 - 2 D. BEN-ISHAI ET. AL. 'Rearrangement in the indan-1,3-dione system'
- SYNTHESIS. no. 3, 1973, STUTTGART DE pages 154 - 5 D. MATTHIES ET. AL. 'Amidoalkierende Derivate des Ninhydrins'
- JOURNAL OF PHARMACEUTICAL SCIENCES vol. 56, no. 6, Juin 1967, WASHINGTON US pages 775 - 6 R.S. VARMA ET. AL. 'Thiosemicarbazones derived from indandione-1,3'
- JOURNAL OF ORGANIC CHEMISTRY. vol. 48, no. 23, 18 Novembre 1983, EASTON US pages 34326 - 9 T.L. LEMKE ET. AL. 'Chemistry of beta-triketones. 1. Structure of schiff base intermediates of 2-acyl-1,3-indandiones'
- JOURNAL OF PHARMACEUTICAL SCIENCES vol. 79, no. 9, Septembre 1990, WASHINGTON US pages 840 - 4 T.L. LEMKE ET. AL. 'Isolation, synthesis and evaluation of a series of indenecarbazates as hypotensive agents'
- CHEMICAL ABSTRACTS, vol. 72, no. 11, 16 Mars 1970, Columbus, Ohio, US; abstract no. 55046r, A. VEVERIS ET. AL. 'New derivatives of 1,3-indanedione thiosemicarbazone' page 384 ;colonne 1 ;
- CHEMICAL ABSTRACTS, vol. 81, no. 19, 11 Novembre 1974, Columbus, Ohio, US; abstract no. 120286s, P. A. CROOKS 'New synthesis of 2-aminoindanes' page 503 ;colonne 1 ;
- CHEMICAL ABSTRACTS, vol. 83, no. 1, 7 Juillet 1975, Columbus, Ohio, US; abstract no. 9584q, L. GEITA ET. AL. '2-acetamido-1,3-indandione and its derivatives' page 793 ;colonne 1 ;

## Description

La présente invention a pour objet de nouveaux dérivés de l'indane-1,2-dione et de l'indane-1,2,3-trione, leurs procédés de préparation et leur application en thérapeutique.

Ces dérivés répondent plus précisément à la formule : 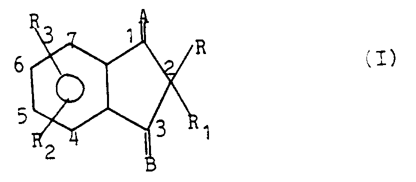
dans laquelle R₂ et R₃ représentent indépendamment l'un de l'autre H, alkoxy en C₁-C₄ ou OH ; et le couple (A, B) prend la valeur :
- (oxygène, oxygène), auquel cas l'un parmi R et R₁ représente OH, halogène, (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène et l'autre représente un groupe choisi parmi les suivants :
   . NHCOR₄ où R₄ = phényle ; alkyle en C₁-C₄ ; ou alkyle en C₁-C₄ substitué par un halogène,
   . NHNHCOR₅ où R₅ = alkoxy en C₁-C₄ ; phényle, phényle substitué par un ou deux groupes choisis parmi les suivants : amino, alkoxy en C₁-C₄, alkyle en C₁-C₄, OH ; thiényle ; furyle ; pyridyle ; indolyl-2-méthyle ; indolyl-3-méthyle ; ou 5-phényl-2-(N-pyrrolyl)thiényle,
   . NHNHCOCONH₂, à l'exclusion des dérivés pour lesquels l'un parmi R et R₁ représente OH ou halogène et l'autre représente NHCOR₄ où R₄ = phényle ou alkyle en C₁-C₄ ; des dérivés pour lesquels l'un parmi R et R₁ représente (alkyl en C₁-C₄)NH et l'autre représente NHCOR₄ où R₄ = alkyle en C₁-C₄ ; et des dérivés pour lesquels l'un parmi R et R₁ représente OH et l'autre représente NHNHCOR₅ où R₅ = pyridyle ou phényle substitué par un groupe alkoxy en C₁-C₄,
      R et R₁ pouvant par ailleurs former ensemble un groupe :
   . =NNHCOR'₅ où R'₅ = R₅, trifluoroacétylaminophényle, acétylaminophényle, pyrazinyle ou pyrrolyle,
   . =NNHCOCONH₂,
   . =NNHR₆ où R₆ = phényle ; phényle substitué par un ou deux groupes choisis parmi alkyle en C₁-C₄, halogène et alkoxy en C₁-C₄ ; méthylsulfonylphényle ; et N-méthylméthanesulfonamidophényle,
   . =N-R₇ où R₇ = phényle ou phényle substitué par un groupe OH,
   . =N-O-CO-R₈ où R₈ = phényle ou phényle substitué par un atome d'halogène, ou
   . =C(CH₃)-NH-NH-CO-R₉ où R₉ = phényle, phénylamino, phényle substitué par un atome d'halogène ou un groupe OH, ou phénylamino substitué par un atome d'halogène ou un groupe OH,
   à l'exception des dérivés pour lesquels R et R₁ forment ensemble un groupe =NNHCOR'₅ où R'₅ = pyridyle ou phényle substitué par alkoxy en C₁-C₄ et des dérivés pour lesquels R et R₁ forment ensemble un groupe =N-NHR₆ où R₆ = phényle ou phényle substitué par un atome d'halogène ou un groupe alkyle ou alkoxy en C₁-C₄ ; ou
- (NNHCXNHR₁₀, oxygène) où X représente oxygène ou soufre et R₁₀ = H, phényle ou phényle substitué par un ou deux groupes choisis parmi OH, CF₃, alkyle en C₁-C₄, alkoxy en C₁-C₄, halogène, méthylènedioxy, acétoxy et hydroxyéthyle, auquel cas R et R₁ forment ensemble un groupe =N-OH.

Entrent dans la portée de la formule (I) ci-dessus :
(i) les composés de formule : 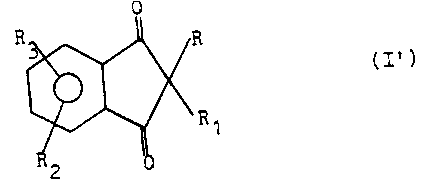 où R, R₁, R₂ et R₃ ont les mêmes significations que dans la formule (I) quand le couple (A, B) y prend la valeur (oxygène, oxygène), et
(ii) les composés de formule : 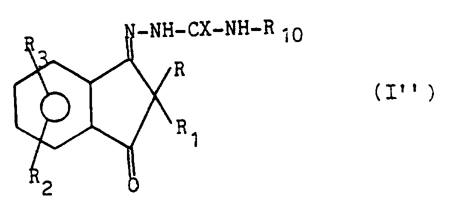 où R, R₁, R₂, R₃, X et R₁₀ ont les mêmes significations que dans la formule (I) quand le couple (A, B) y prend la signification (=NNHCXNHR₁₀, oxygène.

Entrent dans la portée de la formule (I) ci-dessus :
(a) les composés de formule : 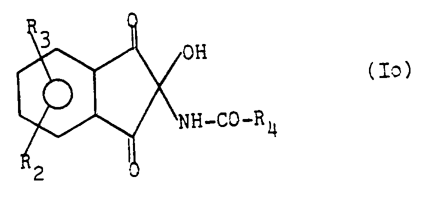 où R₄ = alkyle en C₁-C₄ substitué par un halogène.
(b) les composés de formule : 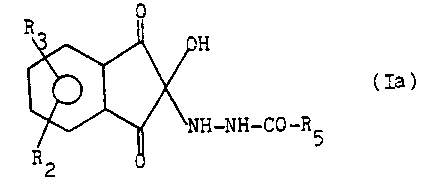 où R₅ ≠ pyridyle et phényle substitué par un groupe alkoxy en C₁-C₄.
(c) les composés de formule : 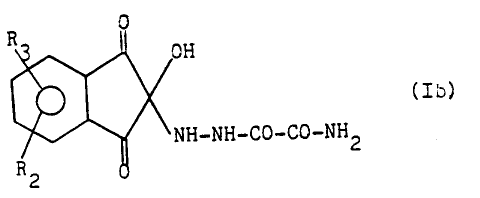
(d) les composés de formule : 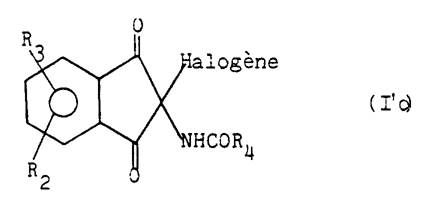 où R₄ = alkyle en C₁-C₄ substitué par un halogène.
(e) les composés de formule : 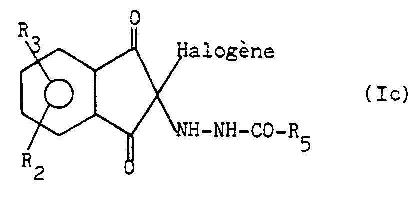
(f) les composés de formule : 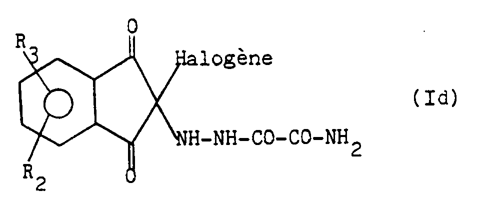
(g) les composés de formule : 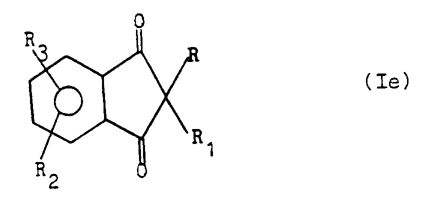 où l'un parmi R et R₁ représente NHCOR₄ et l'autre représente (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène, à l'exception des composés pour lesquels l'un parmi R et R₁ représente NHCOR₄ où R₄ = alkyle en C₁-C₄ et l'autre représente (alkyl en C₁-C₄ )NH.
(h) les composés de formule : 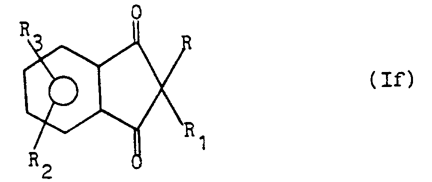 où l'un parmi R et R₁ représente NHNHCOR₅ et l'autre représente (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène.
(i) les composés de formule : 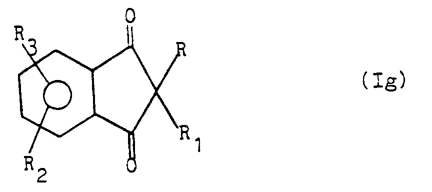 où l'un parmi R et R₁ représente NHNHCOCONH₂ et l'autre représente (alkyl en C₁-C₄)NH, morpholino(alkyl en C₁-C₄)NH, 1-(pyridiyl)-4-pipérazino ou 1-(phényl)-4-pipérazino dont le noyau phényle est éventuellement substitué par un halogène.
(j) les composés de formule : 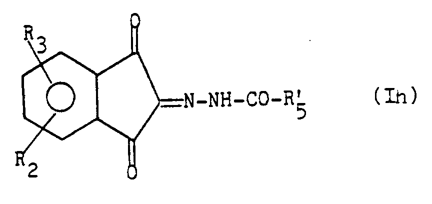 à l'exception de ceux pour lesquels R'₅ = phényle substitué par alkoxy en C₁-C₄ ; ou pyridyle.
(k) les composés de formule : 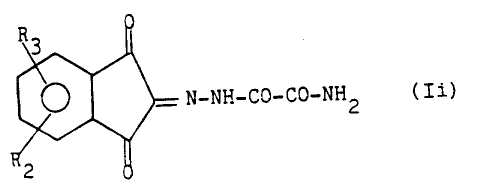
(l) les composés de formule : 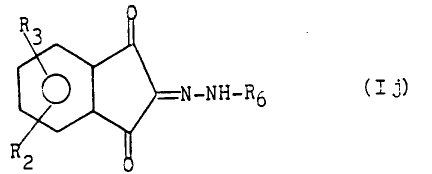 à l'exception des composés pour lesquels R₆ = phényle ou phényle substitué par un atome d'halogène ou un groupe alkyle ou alkoxy en C₁-C₄.
(m) les composés de formule : 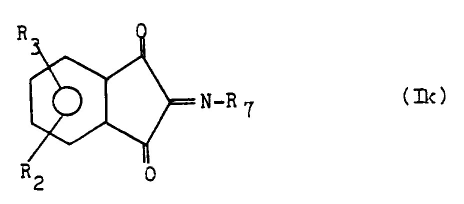
(n) les composés de formule : 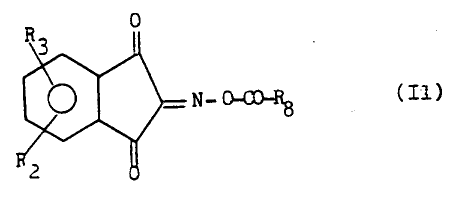
(o) les composés de formule : 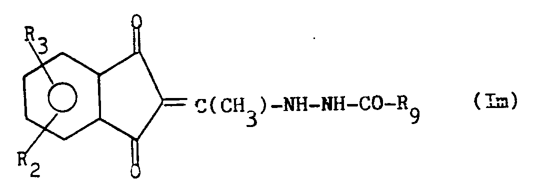
(p) les composés de formule : 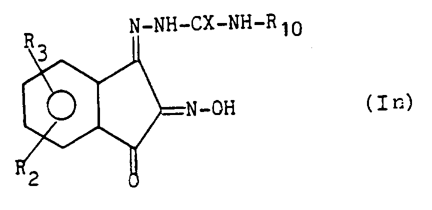 les symboles R₂, R₃, R₅, R'₅, R₆, R₇, R₈, R₉, R₁₀ et X ayant les mêmes significations que dans la formule (I).

Dans les formules (I') et (I'') et (Ia) à (In) ci-dessus, R₂ est notamment en position 5 et R₃ en position 6.

On ajoutera également que dans les formules (I') et (I'') et (Ia) à (In), le couple (R₂, R₃) peut notamment être (H, H), (5-OCH₃,H), (5-OH, H) ou (5-OCH₃, 6-OCH₃).

La présente invention comprend également les sels des composés salifiables parmi ceux décrits ci-dessus. Ces sels comprennent les sels d'addition d'acides minéraux tels que l'acide chlorhydrique, bromhydrique, sulfurique ou phosphorique, et les sels d'addition d'acides organiques tels que l'acide acétique, propionique, oxalique ou citrique.

L'invention englobe en outre tous les stéréoisomères possibles des dérivés de formule (I) et les mélanges de tels stéréoisomères, ainsi que les métabolites de ces dérivés.

Entrent également dans la portée de la présente invention, les procédés de préparation des dérivés de formule (I). Ces procédés font l'objet des schémas 1 à 5 ci-après dans lesquels les symboles R à R₁₀ et X ont, sauf autre indication, les mêmes significations que dans la formule (I).

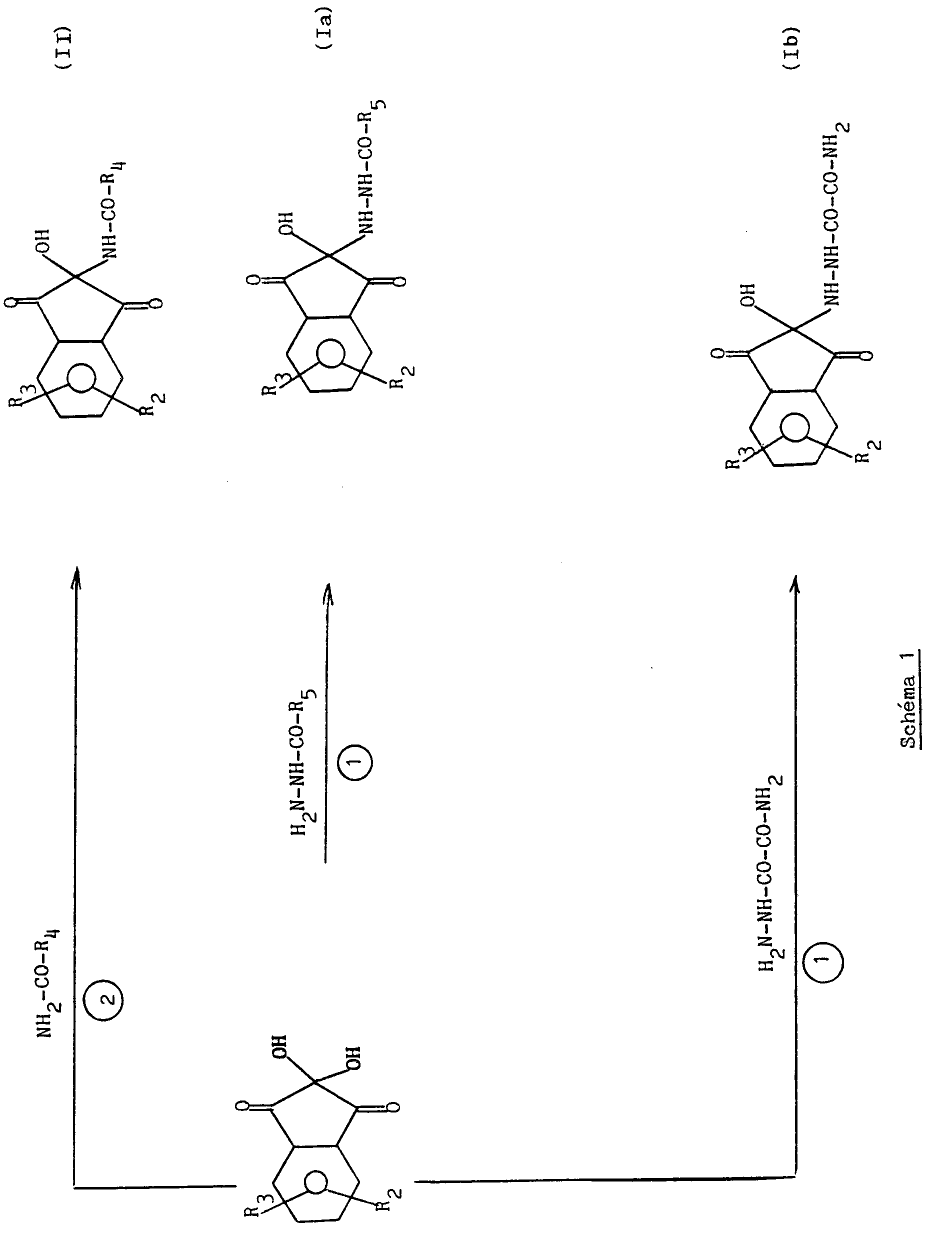

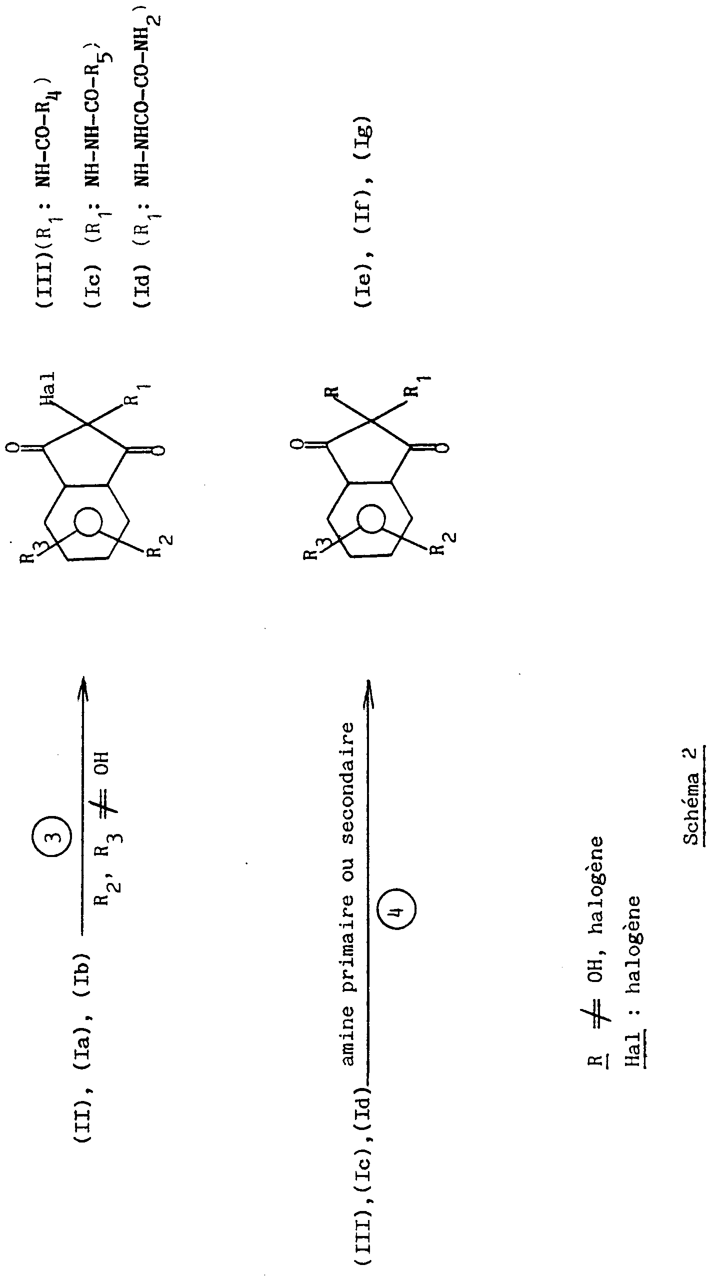

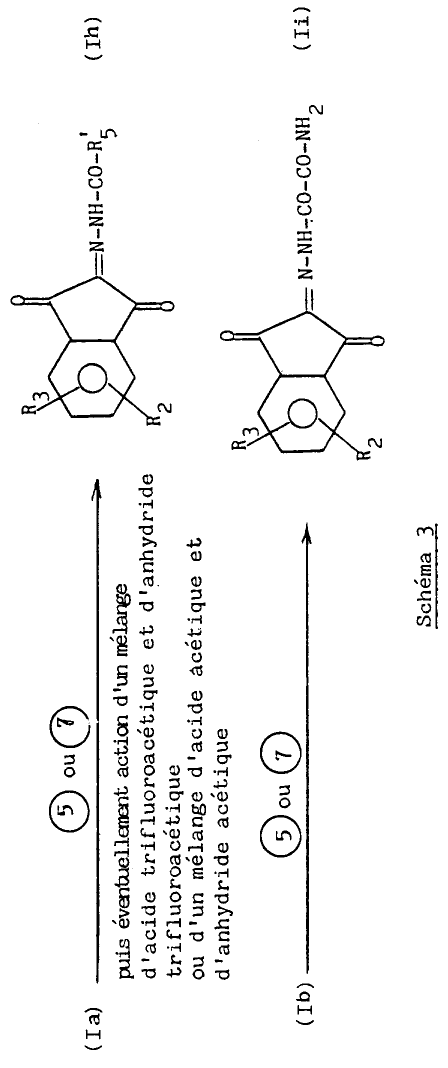

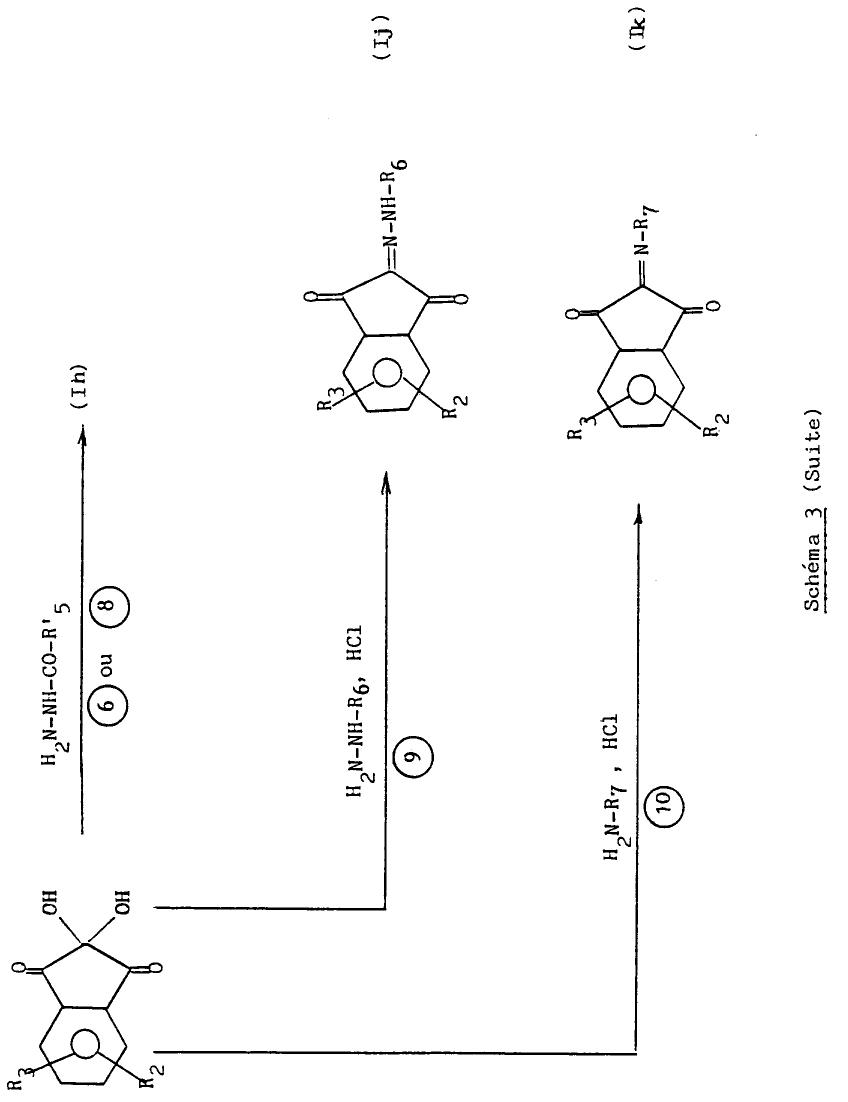

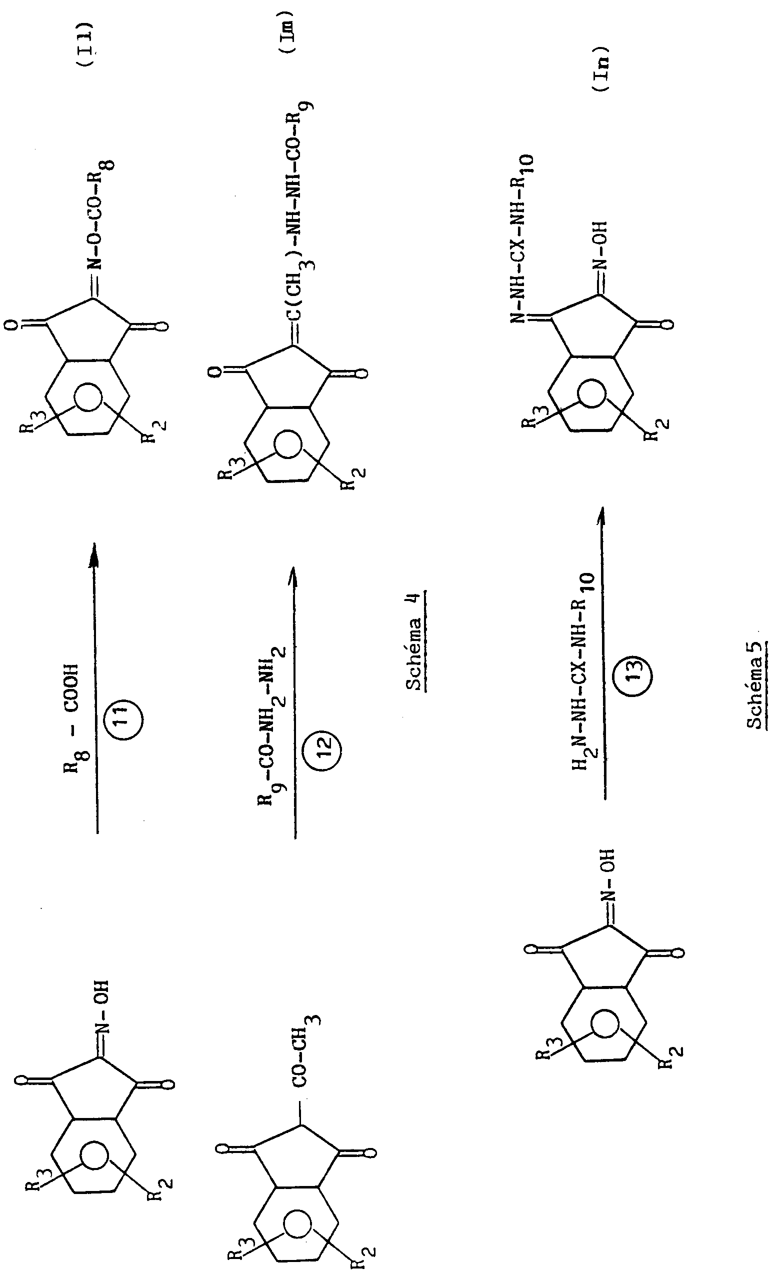

Dans les schémas ci-dessus, ① à ⑤ désignent les méthodes utilisées et ont les significations suivantes :
① Condensation dans un solvant tel que l'éthanol, à chaud, de préférence au reflux.
② Condensation dans un solvant tel que le benzène, à chaud, de préférence au reflux.
③ Réaction avec un agent halogénant, notamment un agent chlorant et de préférence le chlorure de thionyle, dans un solvant tel que le THF, à chaud, de préférence au reflux.
④ Condensation avec une amine primaire [(alkyle en C₁-C₄)NH₂ ou N-morpholino(alkyl en C₁-C₄)NH₂] ou une amine secondaire [1-(pyridyl)-4-pipérazine ou 1-(phényl)-4- pipérazine dont le noyau phényle est éventuellement substitué par un atome d'halogène], dans un solvant tel que l'éther diéthylique, en présence d'une base telle que la triéthylamine.
⑤ Traitement à chaud, de préférence au reflux, dans l'éthanol et en présence d'HCl concentré.
⑥ Condensation à chaud, de préférence au reflux, dans un solvant tel que l'éthanol et en présence d'HCl concentré.
⑦ Chauffage de préférence au reflux, d'une suspension de (Ia) ou (Ib) dans l'acétonitrile.
⑧ Condensation à chaud, de préférence au reflux, dans un solvant tel que l'éthanol.
⑨ Condensation à chaud, de préférence au reflux, dans un solvant tel que l'éthanol aqueux.
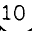 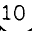 Condensation à chaud, de préférence au reflux, dans un solvant tel que l'éthanol aqueux.
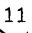 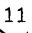 Condensation du chloroformate d'isobutyle sur R₈-COOH, notamment dans un solvant tel que l'acétate d'éthyle et en présence d'une base telle que la N-méthylmorpholine ; l'anhydride mixte obtenu est ensuite condensé avec l'hydroxyimine.
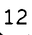 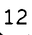 Condensation, notamment dans un solvant, de préférence l'éthanol, de préférence à reflux.
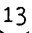 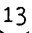 Condensation du semicarbazide ou thiosemicarbazide, sous forme de chlorhydrate, notamment dans un solvant tel que l'éthanol aqueux, avantageusement à chaud et de préférence au reflux.
On ajoutera que les sels des composés salifiables parmi ceux de formule (I) pourront par exemple être obtenus par action d'un acide sur le composé salifiable en solution dans un solvant organique approprié.

### Les préparations suivantes sont données à titre d'exemples pour illustrer l'invention.

### A/ Préparation des matières premières

### Exemple a

### 5-Hydroxyindane-1,3-dione

A une suspension de 0,01 mole de 5-acétoxy-2-carbéthoxy-3-hydroxy-1-indanone dans 80 ml d'eau, on ajoute 4 ml d'acide sulfurique 10N. Le mélange réactionnel est porté au reflux pendant 15 minutes. L'insoluble est filtré à chaud et le précipité jaune formé après refroidissement est essoré, lavé à l'eau glacée puis séché. Rdt. : 85 % ; F : 208° C; IR : ν OH = 3250 cm⁻¹, ν CO = 1730 et 1680 cm⁻¹

### Exemple b

### 2-Bromo-5-hydroxyindane-1,3-dione

A une solution de 0,02 mole de 5-hydroxyindane-1,3-dione dans 60 ml de chloroforme, on ajoute goutte à goutte 0,02 moles de brome. Le mélange réactionnel est agité à 50° C pendant 30 minutes et le précipité obtenu après évaporation du solvant est recristallisé.
Rd. : 60 % ; F : 194° C; IR : ν OH = 3380 cm⁻¹, ν CO = 1740 et 1710 cm⁻¹ Solvant de recristallisation : mélange (V/V) éther éthylique/éther de pétrole.

### Exemple c

### 5-Hydroxyindane-1,2,3-trione, monohydrate

Une solution de 0,01 mole de 2-bromo-5-hydroxyindane-1,3-dione dans 10 ml de diméthylsulfoxyde est chauffée à 80° C pendant 30 minutes. On ajoute 50 ml d'une solution d'acide chlorhydrique 1N, puis on prolonge le chauffage 30 minutes. Après refroidissement, la suspension huileuse obtenue est extraite à l'éther éthylique. La phase organique est lavée à l'eau et séchée au sulfate de magnésium.

Le précipité jaune obtenu après évaporation du solvant est recristallisé dans l'eau. Rdt. : 45 % ; F : > 265° C; IR : ν OH = 3340 cm⁻¹, ν CO = 1750 et 1710 cm⁻¹.

### Exemple d

### 5,6-Diméthoxyindane-1,2,3-trione, monohydrate

A une solution de 0,03 mode de 5,6-diméthoxy-1-indanone dans 100 ml de dioxanne, on ajoute 0,06 mode d'oxyde de sélénium en solution dans 2,5 ml d'eau. Le mélange réactionnel est porté au reflux pendant 7 heures et le résidu obtenu après évaporation du solvant est dissous dans l'acétate d'éthyle. La phase organique est lavée plusieurs fois à l'eau, séchée sur sulfate de magnésium, puis décolorée.

Le précipité obtenu après évaporation du solvant est recristallisé dans l'éther éthylique.
Rdt. : 50 % ; F : 170° C ; IR : ν CO = 1730 et 1700 cm⁻¹

### Exemple e

### 6-Hydroxy-5-méthoxyindane-1,2,3-trione, monohydrate

A une solution de 0,03 mode de 6-hydroxy-5-méthoxy-1-indanone dans 100 ml de dioxane, on ajoute 0,06 mode d'oxyde de sélénium en solution dans 2,5 ml d'eau. Le mélange réactionnel est porté au reflux pendant 7 heures et le résidu obtenu après évaporation du solvant est dissous dans l'acétate d'éthyle. La phase organique est lavée plusieurs fois à l'eau, séchée sur sulfate de magnésium puis décolorée.

Le précipité obtenu après évaporation du solvant est recristallisé dans l'eau.
Rdt. : 50 % ; F : > 265° C ; IR : ν CO = 1740 et 1700 cm⁻¹

### Exemple f

### 2-Oximino-5-hydroxyindane-1,3-dione

A une suspension de 0,01 mode de 5-hydroxyindane-1,3-dione dans 6 ml d'une solution d'acide sulfurique 2N, on ajoute goutte à goutte en maintenant la température à 5° C, 0,02 mode de nitrite de sodium en solution dans 8 ml d'eau. Le mélange réactionnel est agité 4 heures à 5° C. L'insoluble est essoré, lavé à l'eau, séché et recristallisé dans l'acétone.
Rdt. : 65 % ; F : 220° C ; IR : ν OH = 3400 et 3260 cm⁻¹, ν CO = 1730 et 1690 cm⁻¹
Dans ce qui suit, on utilise indifféremment les noms ninhydrine, indane-1,2,3-trione monohydrate et 2,2-dihydroxy-1,3-dioxo-2H-indène.

### B/ Préparation des composés de formules (Ia)-(Id), (II) et (III) Méthode ①

A une solution de 0,01 mode de ninhydrine dans 20 ml d'éthanol, on ajoute 0,01 mole de H₂N-NH-CO-R₅ ou H₂N-NH-CO-CO-NH₂ en solution dans 20 ml d'éthanol.

Le mélange réactionnel est chauffé 60° C pendant 5 à 15 minutes et le précipité blanc formé est essoré, lavé à l'éther éthylique puis séché.

### Méthode ②

A une solution de 0,04 mole de ninhydrine dans 40 ml de benzène, on ajoute 0,04 mode de NH₂-CO-R₄. Le mélange réactionnel est porté au reflux pendant une heure. Le précipité blanc formé après refroidissement dans un bain de glace est essoré et dissous dans 150 ml d'éther éthylique. La phase organique est lavée trois fois avec 100 ml d'eau et séchée sur sulfate de magnésium. Les cristaux blancs formés après évaporation du solvant sont recristallisés.

### Méthode ③

A une solution de 6 g (0,02 mode) de 2-benzamido-2-hydroxyindane-1,3-dione dans 20 ml de tétrahydrofurane, on ajoute goutte à goutte 3,5 ml (0,04 mode) de chlorure de thionyle. Le mélange réactionnel est porté au reflux pendant une heure. L'huile résiduelle obtenue après évaporation de l'excès de chlorure de thionyle et de tétrahydrofurane, cristallise après un lavage à l'éther de pétrole.

### Exemple 1

### 2-Hydroxy-2-méthoxycarbonylhydrazinoindane-1,3-dione

### Méthode ①

Matières premières : Indane-1,2,3-trione monohydrate Méthoxycarbonylhydrazine
Rdt. : 90 % ; F : 188° C; IR : ν OH = 3360 cm⁻¹, ν NH = 3260 cm⁻¹, ν CO = 1760 et 1700 cm⁻¹.

### Exemple 2

### 2-Hydroxy-2-méthoxycarbonylhydrazino-5-méthoxyindane-1,3-dione

### Méthode ①

Matières premières : 5-Méthoxyindane-1,2,3-trione monohydrate Méthoxycarbonylhydrazine
Rdt. : 81 % ; F : 190° C ; IR : ν OH = 3360 cm⁻¹, ν NH = 3270 cm⁻¹, ν CO = 1760 et 1720 cm⁻¹

### Exemple 3

### 2-Benzoylhydrazino-2-hydroxy-indane-1,3-dione

### Méthode ①

Matières premières : Indane-1,2,3-trione monohydrate Benzoylhydrazine
Rdt. : 93 % ; F : 188° C ; 1R : ν OH = 3320 cm⁻¹, ν NH = 3240 cm⁻¹, ν CO = 1750, 1720 et 1640 cm⁻¹

### Exemple 4

### 2-Benzoylhydrazino-2-hydroxy-5-méthoxyindane-1,3-dione

### Méthode ①

Matières premières : 5-Méthoxyindane-1,2,3-trione monohydrate Benzoylhydrazine
Rdt. : 93 % ; F : 180° C; IR : ν OH = 3350 cm⁻¹, ν NH = 3250 cm⁻¹, ν CO = 1750, 1720 et 1650 cm⁻¹

### Exemple 5

### 2-Hydroxy-2-(3-méthoxybenzoylhydrazino)-5-méthoxyindane-1,3-dione

### Méthode ①

Matières premières : 5-Méthoxyindane-1,2,3-trione monohydrate 3-Méthoxybenzoylhydrazine
Rdt. : 85 % ; F : 178° C; IR : ν OH = 3360 cm⁻¹, ν NH = 3250 cm⁻¹, ν CO = 1760, 1720 et 1650 cm⁻¹

### Exemple 6

### 2-Hydroxy-2-(2-méthylbenzoylhydrazino)-indane-1,3-dione

### Méthode ①

Matières premières : Indane-1,2,3-trione monohydrate 2-Méthylbenzoylhydrazine
Rdt. : 98 % ; F : 192° C ; IR : ν OH = 3300 cm⁻¹, ν NH = 3220 cm⁻¹, ν CO = 1750, 1720 et 1630 cm⁻¹

### Exemple 7

### 2-Hydroxy-2-(3-méthylbenzoylhydrazino)-indane-1,3-dione

### Méthode ①

Matières premières : Indane-1,2,3-trione monohydrate 3-Méthylbenzoylhydrazine
Rdt. : 98 % ; F : 184° C; IR : ν OH = 3360 cm⁻¹, ν NH = 3160 cm⁻¹, ν CO = 1740, 1700 et 1640 cm⁻¹

### Exemple 8

### 2-Hydroxy-2-(4-méthylbenzoylhydrazino)-indane-1,3-dione

### Méthode ①

Matières premières : Indane-1,2,3-trione monohydrate 4-Méthylbenzoylhydrazine
Rdt. : 98 % ; F : 200° C ; IR : ν OH = 3340 cm⁻¹, ν NH = 3220 cm⁻¹, ν CO = 1750, 1720 et 1630 cm⁻¹

### Exemple 9

### 2-Hydroxy-2-(2-hydroxybenzoylhydrazino)-indane-1,3-dione

### Méthode ①

Matières premières : Indane-1,2,3-trione monohydrate 2-Hydroxybenzoyldhydrazine
Rdt. : 98 % ; F : 170° C; IR : ν OH = 3350 cm⁻¹, ν NH = 3220 cm⁻¹, ν CO = 1760, 1720 et 1640 cm⁻¹

### Exemple 10

### 2-Hydroxy-2-(3-hydroxybenzoylhydrazino)-indane-1,3-dione

### Méthode ①

Matières premières : Indane-1,2,3-trione monohydrate 3-Hydroxybenzoylhydrazine
Rdt. : 98 % ; F : 210° C ; IR : ν OH = 3350 cm⁻¹, ν NH = 3220 cm⁻¹, ν CO = 1760, 1720 et 1640 cm⁻¹

### Exemple 11

### 2-(3,4-Diméthoxybenzoylhydrazino)-2-hydroxyindane-1,3 dione

### Méthode ①

Matières premières : Indane-1,2,3-trione monohydrate 3,4-Diméthoxybenzoylhydrazine
Rdt. : 90 % ; F : 210° C ; IR : ν OH = 3360 cm⁻¹, ν NH = 3250 cm⁻¹, ν CO = 1760, 1730 et 1630 cm⁻¹

### Exemple 12

### 2-Hydroxy-2-(2-hydroxy-3-méthylbenzoylhydrazino)-indane-1,3-dione

### Méthode ①

Matières premières : Indane-1,2,3-trione monohydrate 2-Hydroxy-3-méthylbenzoylhydrazine
Rdt. : 98 % ; F : 216° C ; IR : ν OH = 3360 cm⁻¹, ν NH = 3240 cm⁻¹, ν CO = 1760, 1720 et 1635 cm⁻¹

### Exemple 13

### 2-Hydroxy-2-(4-hydroxy-3-méthoxybenzoylhydrazino)-indane-1,3-dione

### Méthode ①

Matières premières : Indane-1,2,3-trione monohydrate 4-Hydroxy-3-méthoxybenzoylhydrazine
Rdt. : 98 % ; F : 236° C ; IR : ν OH = 3350 cm⁻¹, ν NH = 3220 cm⁻¹, ν CO = 1750, 1720 et 1635 cm⁻¹

### Exemple 14

### 2-Hydroxy-2-(5-phényl-2-N-pyrrolyl)thiophène-3-carbonylhydrazino)-indane-1,3-dione

### Méthode ①

Matières premières : Indane-1,2,3-trione monohydrate 5-Phényl-2-(N-pyrrolyl)thiophène-3 carbonylhydrazine
Rdt. : 90 % ; F : 200° C; IR : ν OH = 3300 cm⁻¹, ν NH = 3200 cm⁻¹, ν CO = 1730, 1700 et 1625 cm⁻¹

### Exemple 15

### 2-Hydroxy-2-oxamoylhydrazinoindane-1,3-dione

### Méthode ①

Matières premières : Indane-1,2,3-trione monohydrate Oxamoylhydrazine
Rdt. : 95 % ; F : 260° C ; IR : ν OH = 3420 cm⁻¹, ν NH = 3300, 3200 et 3100 cm⁻¹, ν CO = 1760, 1720 et 1670 cm⁻¹

### Exemple 16

### 2-Hydroxy-2-(indole-3-acéthylhydrazino)-indane-1,3-dione

### Méthode ①

Matières premières : Indane-1,2,3-trione monohydrate Indole-3-acéthylhydrazine
Rdt. : 90 % ; F : 204° C; IR : ν OH = 3350 cm⁻¹, ν NH = 3225 cm⁻¹, ν CO = 1720 et 1680 cm⁻¹

### Exemple 17

### 2-Chloroacétamido-2-hydroxyindane-1,3-dione

### Méthode ②

Matières premières : Indane-1,2,3-trione monohydrate 2-Chloroacétamide
Rdt. : 85 % ; F : 164° C; IR : ν OH = 3380 cm⁻¹, ν NH = 3180 cm⁻¹, ν CO = 1765, 1730 et 1640 cm⁻¹
Solvant de recristallisation : éther éthylique

### Exemple 18

### 2-(4-Aminobenzoylhydrazino)-2-hydroxy-indane-1,3-dione

### Méthode ①

Matières premières : Ninhydrine ; 4-aminobenzoylhydrazine
Rdt. : 80 % ; F : 220° C ; IR : ν OH = 3440 cm⁻¹, ν NH = 3340 et 3220 cm⁻¹, ν CO = 1750 et 1720 cm⁻¹

### C/ Préparation des composés de formules (Ie)-(Ig)

### Méthode ④

A une solution de 0,007 mode de 2-benzamido-2-chloroindane-1,3-dione dans 60 ml d'éther éthylique, on ajoute 1 ml de triéthylamine. On laisse agiter pendant 5 minutes, puis on ajoute 0,007 mode d'amine. Le mélange réactionnel est agité à température ambiante pendant 2 heures. Le précipité formé est lavé plusieurs fois à l'eau, séché et recristallisé.

### Exemple 19

### 2-(2-Aminoéthylmorpholino)-2-benzamidoindane-1,3-dione

Matières premières : 2-Benzamido-2-chloroindane-1,3-dione 4-(2-Aminoéthyl)-morpholine
Rdt. : 85 % ; F : 158° C ; IR : ν NH = 3120 cm⁻¹, ν CO = 1760, 1720 et 1630 cm⁻¹
Solvant de recristallisation : éther éthylique.

### Exemple 20

### 2-Benzamido-2-[1-(2-pyridyl)-pipérazino]indane-1,3-dione

Matières premières : 2-Benzamido-2-chloroindane-1,3-dione 1-(2-Pyridyl)-pipérazine
Rdt. : 70 % ; F : 186° C ; IR : ν NH = 3300 cm⁻¹, ν CO = 1750 et 1720 cm⁻¹
Solvant de recristallisation : éther éthylique.

### Exemple 21

### 2-Benzamido-2-[1-(4-fluorophényl)-pipérazino]indane-1,3-dione

Matières premières : 2-Benzamido-2-chloroindane-1,3-dione 1-(4-Fluorophényl)-pipérazine
Rdt. : 65 % ; F : 162° C ; IR : ν NH = 3200 cm⁻¹, ν CO = 1750, 1720 et 1630 cm⁻¹
Solvant de recristallisation : éther éthylique.

### D/ Préparation des composés de formules (Ih) et (Ii)

### Méthode ⑤

A une suspension de 0,008 mode de composé (Ia) ou (Ib) dans 100 ml d'éthanol, on ajoute 5 gouttes d'acide chlorhydrique concentré (10N). Le mélange réactionnel est porté au reflux pendant 30 minutes. Le précipité jaune formé est essoré à froid, séché et recristallisé.

### Méthode ⑥

A une solution de 0,01 mode de ninhydrine dans 50 ml d'éthanol, on ajoute successivement, 0,01 mode d'hydrazide correspondant en solution dans 50 ml d'éthanol et 5 gouttes d'acide chlorhydrique concentré (10N).

Le mélange réactionnel est porté au reflux pendant 30 minutes ; laisser refroidir à environ 50° C et de précipité jaune obtenu est essoré, séché et recristallisé.

### Méthode ⑦

Une suspension de 1,5 g de composé (Ia) ou (Ib) dans 100 ml d'acétonitrile est portée au reflux pendant 30 minutes. La solution obtenue est maintenue au reflux pendant 30 minutes, jusqu'à l'apparition d'un précipité jaune que l'on essore après refroidissement.

### Méthode ⑧

A une solution de 0,01 mole de ninhydride dans 60 ml d'éthanol, on ajoute 0,01 mole d'hydrazide. Le mélange réactionnel est porté au reflux pendant 2 heures. Le précipité orangé obtenu est essoré, séché et recristallisé.

### Exemple 22

### 2-méthoxycarbonylhydrazonoindane-1,3-dione

### Méthode ⑤

Matière première : 2-Hydroxy-2-méthoxycarbonylhydrazinoindane-1,3-dione
Rdt. : 80 % ; F : 206° C; IR : ν NH = 3220 cm⁻¹, ν CO = 1775, 1720 et 1680 cm⁻¹
Solvant de recristallisation : acétonitrile.

### Exemple 23

### 2-méthoxycarbonylhydrazono-5-méthoxyindane-1,3-dione

### Méthode ⑤

Matière première : 2-Hydroxy-2-méthoxycarbonylhydrazino-5-méthoxyindane-1,3-dione
Rdt. : 81 % ; F : 190° C ; IR : ν NH = 3270 cm⁻¹, ν CO = 1760 et 1720 cm⁻¹
Solvant de recristallisation : acétate d'éthyle.

### Exemple 24

### 2-Benzoylhydrazonoindane-1,3-dione

### Méthode ⑤

Matière première : 2-Benzoylhydrazino-2-hydroxyindane-1,3-dione
Rdt. : 92 % ; F : 202° C; IR : ν NH = 3250 cm⁻¹, ν CO = 1730 et 1680 cm⁻¹
Solvant de recristallisation : acétonitrile.

### Exemple 25

### 2-Benzoylhydrazono-5-méthoxyindane-1,3-dione

### Méthode ⑤

Matière première : 2-Benzoythydrazino-2-hydroxy-5-méthoxyindane-1,3-dione
Rdt. : 98 % ; F : 218° C ; IR : ν NH = 3250 cm⁻¹, ν CO = 1735 et 1680 cm⁻¹
Solvant de recristallisation : acétate d'éthyle.

### Exemple 26

### 2-(2-Méthylbenzoylhydrazono)-indane-1,3-dione

### Méthode ⑤

Matière première : 2-Hydroxy-2-(2-méthylbenzoylhydrazino)-indane-1,3-dione
Rdt. : 98 % ; F : 232° C; IR : ν NH = 3230 cm⁻¹, ν CO = 1720 et 1675 cm⁻¹
Solvant de recristallisation : méthanol.

### Exemple 27

### 2-(3-Méthylbenzoylhydrazono)-indane-1,3-dione

### Méthode ⑥

Matières premières : Indane-1,2,3-trione monohydrate 3-Méthylbenzoylhydrazine
Rdt. 95 % ; F : 192° C ; IR : ν NH = 3230 cm⁻¹, ν CO = 1720 et 1675 cm⁻¹
Solvant de recristallisation : acétate d'éthyle.

### Exemple 28

### 2-(4-Méthylbenzoylhydrazono)-indane-1,3-dione

### Méthode ⑥

Matières premières : Indane-1,2,3-trione monohydrate 4-Méthylbenzoylhydrazine
Rdt. : 95 % ; F : 196° C ; IR : ν NH = 3230 cm⁻¹, ν CO = 1720 et 1675 cm⁻¹
Solvant de recristallisation : éthanol.

### Exemple 29

### 2-(2-Hydroxybenzoylhydrazono)-indane-1,3-dione

### Méthode ⑤

Matière première : 2-Hydroxy-2-(2-hydroxybenzoylhydrazino)-indane-1,3-dione
Rdt. : 98 % ; F : 190° C ; IR : ν OH = 3380 cm⁻¹, νNH = 3140 cm⁻¹, ν CO = 1730, 1690 et 1655 cm⁻¹
Solvant de recristallisation : acétate d'éthyle.

### Exemple 30

### 2-(3-Hydroxybenzoylhydrazono)-indane-1,3-dione

### Méthode ⑤

Matière première : 2-Hydroxy-2-(3-hydroxybenzoylhydrazino)-indane-1,3-dione
Rdt. : 90 % ; F : 262° C; IR : ν OH = 3340 cm⁻¹, ν NH = 3320 cm⁻¹, ν CO = 1730, et 1675 cm⁻¹
Solvant de recristallisation : acétate d'éthyle.

### Exemple 31

### 2-(4-Hydroxybenzoylhydrazono)-indane-1,3-dione

### Méthode ⑥

Matières premières : Indane-1,2,3-trione monohydrate 4-Hydroxybenzoylhydrazine
Rdt. : 90 % ; F : > 265° C ; IR : ν OH/NH = 3330 cm⁻¹, ν CO = 1730 et 1680 cm⁻¹
Solvant de recristallisation : acétonitrile.

### Exemple 32

### 2-(3,4-Dihydroxybenzoylhydrazono)-indane-1,3-dione

### Méthode ⑥

Matières premières : Indane-1,2,3-trione monohydrate Chlorhydrate de 3,4-dihydroxybenzhydrazine
Rdt. : 70 % ; F : > 260° C; IR : ν OH = 3320 cm⁻¹, ν NH = 3250 cm⁻¹, ν CO = 1730 et 1675 cm⁻¹
Solvant de recristallisation : éthanol.

### Exemple 33

### 2-(5-Phényl-2-(N-pyrrolyl)thiophène-3-carbonylhydrazono)-indane-1,3-dione

### Méthode ⑤

Matière première : 2-Hydroxy-2-(5-phényl-2-(N-pyrrolyl)-thiophène-3-carbonylhydrazino)-indane-1,3-dione
Rdt. : 83 % ; F : 198° C ; IR : ν NH = 3230 cm⁻¹, ν CO = 1715 et 1660 cm⁻¹
Solvant de recristallisation : acétate d'éthyle.

### Exemple 34

### 2-Oxamoylhydrazonoindane-1,3-dione

### Méthode ⑤

Matière première : 2-Hydroxy-2-oxamoylhydrazinoindane-1,3-dione
Rdt. : 70 % ; F : > 265° C; IR : ν OH = 3440 cm⁻¹, ν NH = 3310 cm⁻¹, ν CO = 1720, 1700 et 1670 cm⁻¹
Solvant de recristallisation : acétate d'éthyle.

### Exemple 35

### 2-(3-Hydroxybenzoylhydrazono)-5-méthoxyindane-1,3-dione

### Méthode ⑥

Matières premières : 5-Méthoxyindane-1,2,3-trione monohydrate 3-Hydroxybenzoylhydrazine
Rdt. : 70 % ; F : 236° C ; IR : ν OH/NH = 3400 cm⁻¹, ν CO = 1745 et 1680 cm⁻¹
Solvant de recristallisation : acétate d'éthyle.

### Exemple 36

### 2-(3,4-Diméthoxybenzoylhydrazono)-indane-1,3-dione

### Méthode ⑤

Matière première : 2-Hydroxy-2-(3,4-diméthoxybenzoylhydrazino)-indane-1,3-dione
Rdt. : 60 % ; F > 265° C; IR : ν NH = 3250 cm⁻¹, ν CO = 1730 et 1680 cm⁻¹
Solvant de recristallisation : acétate d'éthyle.

### Exemple 37

### 2-(2-Hydroxy-3-méthylbenzoylhydrazono)-indane-1,3-dione

### Méthode ⑤

Matièrepremière:2-Hydroxy-2-(2-hydroxy-3-méthylbenzoylhydrazino)-indane-1,3-dione
Rdt. : 75 % ; F : > 265° C; IR : ν NH = 3250 cm⁻¹, ν CO = 1730 et 1680 cm⁻¹
Précipité lavé à l'eau.

### Exemple 38

### 2-(4-Hydroxy-3-méthoxybenzoylhydrazono)-indane-1,3-dione

### Méthode ⑥

Matières premières : Indane-1,2,3-trione monohydrate 4-Hydroxy-3-méthoxybenzoylhydrazine
Rdt. : 70 % ; F : 264° C; IR : ν OH = 3330 cm⁻¹, ν NH = 3250 cm⁻¹, ν CO = 1730 et 1675 cm⁻¹
Solvant de recristallisation : dioxane.

### Exemple 39

### 2-(2-Thénoylhydrazono)-indane-1,3-dione

### Méthode ⑧

Matières premières : Indane-1,2,3-trione monohydrate 2-Thénoylhydrazine
Rdt. : 80 % ; F : 182° C; IR : ν NH = 3200 cm⁻¹, ν CO = 1720 et 1670 cm⁻¹
Solvant de recristallisation : éthanol.

### Exemple 40

### 2-(2-Furoylhydrazono)-indane-1,3-dione

### Méthode

Matières premières : Indane-1,2,3-trione monohydrate 2-Furoylhydrazine
Rdt. : 70 % ; F : 222° C ; IR : ν NH = 3200 cm⁻¹, ν CO = 1720 et 1670 cm⁻¹
Solvant de recristallisation : éthanol

### Exemple 41

### 2-(4-Aminobenzoylhydrazono)-indane-1,3-dione

### Méthodes ⑤ et ⑥

Matières premières : Indane-1,2,3-trione monohydrate Chlorhydrate de 4-aminobenzhydrazine
Rdt. : 80 % ; F : > 260° C ; IR : ν NH = 3340 et 3220 cm⁻¹, ν CO = 1730 et 1680 cm⁻¹
Solvant de recristallisation : acétonitrile.

### Exemple 42

### 2-(4-Trifluoroacétylaminobenzoylhydrazono)-indane-1,3-dione

On ajoute à un mélange de 8 ml d'anhydride trifluoroacétique et 8 ml d'acide trifluoroacétique, 1 g(0,003 mole) de 2-(4-aminobenzoylhydrazono)-indane-1,3-dione. Le mélange réactionnel est porté au reflux pendant 10 minutes et le précipité jaune obtenu après refroidissement est lavé plusieurs fois à l'eau, essoré, séché et recristallisé.
Rdt. : 75 % ; F : > 260° C ; IR : ν NH = 3220 cm⁻¹, ν CO = 1740 et 1690 cm⁻¹
Solvant de recristallisation : acétate d'éthyle.

### Exemple 43

### 2-(4-Acétylaminobenzoylhydrazono)-indane-1,3-dione

On ajoute à un mélange de 8 ml d'anhydride acétique et 8 ml d'acide acétique, 1 g (0,003mole) de 2-(4-aminobenzoylhydrazono)-indane-1,3-dione. Le mélange réactionnel est agité à température ambiante pendant 30 minutes et le précipité jaune obtenu après refroidissement est lavé plusieurs fois à l'eau, essoré, séché et recristallisé.
Rdt. : 60 % ; F : > 260° C ; IR : ν NH = 3220 cm⁻¹, ν CO = 1740 et 1690 cm⁻¹ Solvant de recristallisation : acétate d'éthyle.

### Exemple 44

### 2-[(2-pyrazinyl)carbonylhydrazono]-indane-1,3-dione

### Méthode ⑥

Matières premières : Indane-1,2,3-trione monohydrate 2-Hydrazinocarbonyl-pyrazine
Rdt. : 95 % ; F : 278° C ; IR (KBr) : ν NH = 3230 cm⁻¹, ν C=O = 1720 et 1700 cm⁻¹
RMN du ¹H (200 MHz, DMSO d₆) : δ (ppm)
14,38 (1s, 1H, NH) ; 9,40 (s, 1H, H-3') ; 9,04 et 8,92 (2dd, 2H, H-5', H-6', 15'-6'=4Hz) ; 8,03 (s, 4H, H-4, H-5, H-6, H-7)
Solvant de recristallisation : dichlorométhane.

### Exemple 45

### 2-[(3-furyl)carbonylhydrazono]-indane-1,3-dione

### Méthode ⑥

Matières premières : Indane-1,2,3-trione monohydrate 3-Hydrazinocarbonyl-furane
Rdt. : 59 % ; F : 180° C ; IR (KBr) : ν NH = 3400 cm⁻¹, ν C=O = 1760 et 1690 cm⁻¹
RMN du ¹H (200MHz, DMSO d₆) : δ (ppm)
12,70 (1s, 1H, NH) ; 8,59 (s, 1H, H-2') ; 8,02 (s, 4H, H-4, H-5, H-6, H-7) ; 7,91 (s, 1H, H-5') ; 6,98 (s, 1H, H-4')
Solvant de recristallisation : acétone.

### Exemple 46

### 2-[(2-pyrrolyl)carbonylhydrazono]-indane-1,3-dione

### Méthode ⑥

Matières premières : Indane-1,2,3-trione monohydrate 2-Hydrazinocarbonyl-pyrrole
Rdt. : 75 % ; F : 235° C ; IR (KBr) : ν NH = 3250 et 3125 cm⁻¹, ν C=O = 1775 et 1700 cm⁻¹
RMN ¹H (200 MHz, DMSO d₆) : δ (ppm)
13,21 (1s, 1H, NHCO); 12,25 (1s, 1H, NH) ; 8,00 (s, 4H, H-4, H-5, H-6, H-7); 7,22 (m, 1H, H-5') ; 7,03 (m, 1H, H-3') ; 6,33 (m, 1H, H-4')
Solvant de recristallisation : acétate d'éthyle.

### Exemple 47

### 2-[(3-pyrrolyl)carbonylhydrazono]-indane-1,3-trione

### Méthode ⑥

Matières premières : Indane-1,2,3-trione monohydrate 3-Hydrazinocarbonyl-pyrrole
Rdt. : 60% ; F : 240° C ; IR (KBr) : ν NH = 3250 et 3125 cm⁻¹, ν C=O = 1775 et 1700 cm⁻¹
RMN ¹H (200 MHz, DMSO d₆) : δ (ppm)
13,28 (1s, 1H, NHCO); 12,00 (1s, 1H, NH) ; 7,95 (s, 4H, H-4, H-5, H-6, H-7) ; 7,10 (m, 1H, H-2') ; 7,00 (m, 1H, H-5') ; 6,25 (m, 1H, H-4')
Solvant de recristallisation : acétate d'éthyle.

### Exemple 48

### 2-[3-thiényl)carbonylhydrazono]-indane-1,3-dione

### Méthode ⑥

Matières premières : Indane-1,2,3-trione monohydrate 3-Hydrazinocarbonyl-thiophène
Rdt. : 75 % ; F : 178° C ; IR (KBr) : ν NH = 3050 cm⁻¹, ν C=O = 1730, 1690 et 1680 cm⁻¹
RMN ¹H (200 MHz, DMSO d₆) : δ (ppm)
13,28 (1s, 1H, NH) ; 7,51 (s, 1H, H-2') ; 8,02 (s, 4H, H-4, H-5, H-6, H-7) ; 7,80 (d, 1H, H-5'), J4'-5'=5Hz) ; 7,61 (d, 1H, H-4', J4'-5'=5Hz)
Solvant de recristallisation : acétate d'éthyle.

### Exemple 49

### 2-[(4-fluorophényl)carbonylhydrazono]-indane-1,3-dione

### Méthode ⑥

Matières premières : Indane-1,2,3-trione monohydrate 4-fluoro-Hydrazinocarbonyl-benzène
Rdt. : 71 % ; F : > 260° C ; IR (KBr) : ν NH = 3120 cm⁻¹, ν C = O = 1730 et 1665 cm⁻¹
RMN du ¹H (200 MHz, DMSO, d₆) : δ (ppm)
13,55 (1s, 1H, NH) ; 8,05 (1s, 6H, H-4, H-5, H-6, H-7, H-3', H-5') ; 7,55 (t, 2H, H-2', H-6', J2'-3' et J5'-6'=6,6Hz)
Solvant de recristallisation : acétate d'éthyle.

### E/ Préparation des composés de formules (Ij) et (Ik)

### Méthode ⑨

A une solution de 0,01 mole de ninhydrine dans 60 ml d'éthanol, on ajoute 0,01 mole de chlorhydrate d'hydrazine correspondant en solution dans 15 ml d'eau.

Le mélange réactionnel et porté au reflux pendant 30 minutes. Le précipité formé est essoré à chaud, séché et recristallisé.

### Exemple 50

### 2-(2,4-Dichlorophénylhydrazono)-indane-1,3-dione

Matières premières : Indane-1,2,3-trione monohydrate 2,4-Dichlorophénylhydrazine
Rdt. : 70 % ; F : 238° C; IR : ν NH = 3200 cm⁻¹, ν CO = 1720 et 1670 cm⁻¹
Solvant de recristallisation : acétate d'éthyle.

### Exemple 51

### 2-(3-Chloro-4-méthylphénylhydrazono)-indane-1,3-dione

Matières premières : Indane-1,2,3-trione monohydrate Chlorhydrate de 3-chloro-4-méthylphénylhydrazine
Rdt. : 70 % ; F : 234° C; IR : ν NH = 3140 cm⁻¹, ν CO = 1710 et 1660 cm⁻¹
Solvant de recristallisation : acétate d'éthyle.

### Exemple 52

### 2-(4-Méthylsulfonylphénylhydrazono)-indane-1,3-dione

### Matières premières : Indane-1,2,3-trione monohydrate Chlorhydrate de 4-méthylsulfonylphénylhydrazine Rdt. : 90 % ; F : > 265° C; IR : ν NH = 3200 cm⁻¹, ν CO = 1720 et 1670 cm⁻¹ Précipité lavé à l'acétate d'éthyle.

### Exemple 53

### 5-hydroxy-2-(4-Méthylsulfonylphénylhydrazono)-indane-1,3-dione

Matières premières : 5-hydroxyindane-1,2,3-trione monohydrate Chlorhydrate de 4-méthylsulfonylphénylhydrazine
Rdt. : 90 % ; F : > 265° C; IR : ν NH/OH = 3320 cm⁻¹, ν CO = 1710 et 1670 cm⁻¹
Précipité lavé à l'acétate d'éthyle.

### Exemple 54

### 2-(4-N-Méthylméthanesulfonamidophénylhydrazono)-indane-1,3-dione

Matières premières : Indane-1,2,3-trione monohydrate Chlorhydrate de 4-(N-méthylméthanesulfonamido)-phénylhydrazine
Rdt. : 90 % ; F : > 265° C; IR : ν NH = 3300 cm⁻¹, ν CO = 1720 et 1675 cm⁻¹
Précipité lavé à l'acétate d'éthyle.

### Exemple 55

### 5-Méthoxy-2-(4-N-Méthylméthanesulfonamidophénylhydrazono)-indane-1,3-dione

Matières premières : 5-Méthoxyindane-1,2,3-trione monohydrate Chlorhydrate de 4-(N-méthylméthanesulfonamido)-phénylhydrazine
Rdt. : 90 % ; F : 236° C; IR : ν NH = 3280 cm⁻¹, ν CO = 1715 et 1670 cm⁻¹
Précipité lavé à l'acétate d'éthyle.

### Exemple 56

### 2-(2-Hydroxyphényl)-iminoindane-1,3-dione

A une solution de 0,01 mole de ninhydrine dans 200 ml d'éthanol, on ajoute 0,01 mole de 2-hydroxyaniline. Le mélange réactionnel est porté au reflux pendant une heure. Le précipité obtenu est essoré, lavé à l'éther éthylique et recristallisé dans l'acétonitrile.

### Méthode 10

Rdt. : 70 % ; F : > 265° C ; IR : ν OH = 3500 cm⁻¹, ν CO = 1750 cm⁻¹

F/ Préparation des composés de formule (II)

Méthode 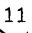

A 34 mmoles d'acide aryl carboxylique en solution dans 68 ml d'acétate d'éthyle, on ajoute à température ambiante 34 mmoles de chloroformate d'isobutyle et 34 mmoles de N-méthylmorpholine, puis goutte à goutte à - 10° C 34 mmoles de 2-hydroxyimino-indane-1,3-dione en solution dans 25 ml de DMF. Après une heure à - 10° C, 15 heures à température ambiante et 5 heures à 40° C, le précipité formé est éliminé par filtration sur verre fritté, lavé par de l'acétate d'éthyle. Le filtrat est ensuite lavé avec une solution saturée d'hydrogénocarbonate de sodium, séché sur sulfate de magnésium, évaporé sous pression réduite. Les cristaux jaunes obtenus sont recristallisés puis séchés une nuit sous vide à 50° C.

### Exemple 57

### 2-phénylcarbonyloxyimino-indane-1,3-dione

Matières premières : 2-hydroxyimino-indane-1,3-dione acide benzène carboxylique
Rdt. : 68 % ; F : 178° C; IR (KBr) : ν C=O = 1780 et 1700 cm⁻¹
RMN ¹H (200 MHz, DMSO, d₆) : δ (ppm)
8,23 (m, 2H, H-2', H-6', J2'-3' et 5'-6'=8,4Hz) ; 8,05 (s, 4H, H-4, H-5, H-6, H-7); 7,78 (m, 1H, H-4') ; 7,70 (m, 2H, H-3', H-5')
Solvant de recristallisation : chloroforme/éther de pétrole 50/50 %.

### Exemple 58

### 2-(4-fluorophényl)carbonyloxyimino-indane-1,3-dione

Matières premières : 2-hydroxyimino-indane-1,3-dione acide 4-fluoro-benzène carboxylique
Rdt. : 75 % ; F : 198° C ; IR (KBr) : ν CO = 1765 et 1700cm⁻¹
RMN ¹H (200 MHz, DMSO d₆) : δ (ppm)
8,30 (m, 2H, H-3', H-5'); 8,05 (s, 4H, H-4, H-5, H-6, H-7) ; 7,53 (t, 2H, H-2', H-6', J2'-3' et 5'-6'=8,9Hz)
Solvant de recristallisation : chloroforme/éther de pétrole (50/50).

G/ Préparation des composés de formule (Im)

Méthode 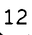

A une solution de 10 mmoles de 2-acétyl-indane-1,3-dione dans 30 ml d'éthanol, on ajoute 10 mmoles d'aryl-aminocarbonylhydrazine en solution dans 60 ml d'éthanol.

Le mélange réactionnel est porté au reflux pendant 30 minutes et le précipité blanc obtenu est essoré à chaud, séché puis recristallisé.

### Exemple 59

### 2-[méthyl(phénylaminocarbonylhydrazino)méthylène]-indane-1,3-dione

Matières premières : 2-acétyl-indane-1,3-dione Phénylaminocarbonylhydrazine
Rdt. : 71 % ; F : 213° C ; IR (KBr) : ν NH = 3280 et 3250 cm⁻¹, ν C=O = 1700, 1660 et 1600 cm⁻¹
RMN ¹H (200 MHz, DMSO d₆) : δ (ppm)
11,55 (s, 1H, NH) ; 9,26 (s, 1H, NH) ; 8,84 (s, 1H, NH) ; 7,60 (s, 4H, H-4, H-5, H-6, H-7) ; 7,41 (d, 2H, H-2', H-6', JH3'-2'=JH6'-5'=8,41Hz) ; 7,22 (t, 2H, H-3', H-5', JH3' 2'=JH6'-5'=8,41 Hz, JH3'-4'=6,92 Hz) ; 6,93 (t, 1H, H-4', JH3'-4'= 6,92 Hz) ; 2,51 (s, 3H, CH₃)
Solvant de recristallisation : acétone

### Exemple 60

### 2-[(4-fluorophényl)aminocarbonylhydrazinométhylméthylène]-indane-1,3-dione

Matières premières : 2-acétyl-indane-1,3-dione (4-Fluorophényl)aminocarbonylhydrazine
Rdt. : 98 % ; F : 213° C; IR (KBr) : ν NH = 3260 et 3240 cm⁻¹, ν CO = 1700, 1660 et 1600 cm⁻¹
RMN ¹H (200 MHz, DMSO, d₆) : δ (ppm)
11,68 (s, 1H, NH) ; 9,44 (s, 1H, NH) ; 9,00 (s, 1H, NH) ; 7,75 (s, 4H, H-4, H-5, H-6, H-7) ; 7,57 (m, 2H, H-3', H-5') ; 7,22 (t, 2H, H-2', H-6', JH2'-3' et 5'-6'=8,9 Hz) ; 2,66 (s, 3H, CH₃)
Solvant de recristallisation : acétone

### Exemple 61

### 2-[(4-hydroxyphényl)carbonylhydrazinométhylméthylène]-indane-1,3-dione

Matières premières : 2-acétyl-indane-1,3-dione (4-hydroxyphényl)carbonylhydrazine
Rdt. : 88 % ; F : 257° C; IR (KBr) : ν NH, OH = 3210 cm⁻¹, ν C=O = 1700, 1640 et 1600 cm⁻¹
RMN du ¹H (200 MHz, DMSO d₆) : δ (ppm)
11,98 (s, 1H, NH) ; 11,16 (s, 1H, NH) ; 10,40 (s, 1H, OH); 7,94 (d, 2H, H-3', H-5', JH2'-3' et 5'-6'=8,41 Hz) ; 7,78 (s, 4H, H-4, H-5, H-6, H-7) ; 7,00 (d, 2H, H-2', H-6', JH2'-3' et 5'-6'=8,41 Hz) ; 2,73 (s, 3H, CH₃)
Solvant de recristallisation : acétone

### H/ Préparation des composés de formule (In)

### Exemple 62

### 2-Oximino-1-(4-phénylsemicarbazono)-3-indanone

A une solution de 0,01 mole de 2-oximino indane-1,3-dione dans 60 ml d'éthanol, on ajoute 0,01 mole de chlorhydrate de 4-phénylsemicarbazide en solution dans 10 ml d'eau. Le mélange réactionnel est porté au reflux pendant 1 heure. Le précipité formé est essoré à chaud, séché et recristallisé dans l'acétonitrile.

Méthode 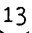

Rdt. : 60 % ; F > 265° C; IR : ν OH = 3340 cm⁻¹, ν NH = 3250 cm⁻¹, ν CO = 1700 et 1680 cm⁻¹
Il convient de préciser que les spectres infrarouges ci-dessus ont été déterminés avec des pastilles KBr.

On donne ci-après les spectres RMN de certains des composés selon l'invention.

On précisera que ces spectres ont été pris sur un appareil de RMN 200 MHz, les composés étant en solution dans le DMSO (D6). Ces spectres, ainsi que ceux donnés ci-dessus, sont décrits selon le protocole suivant: déplacement (δ) en ppm, forme du signal, nombre de protons, nature des protons, constantes de couplage s'il y a lieu.

La numérotation des protons aromatiques de l'indane est comme suit: 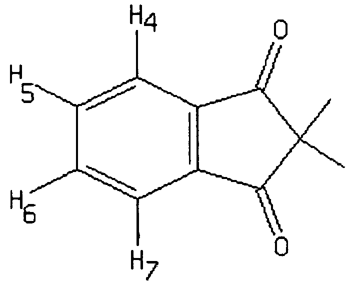
Dans le cas où R et R₁ comportent un hétérocycle ou forment un groupe comportant un hétérocycle, la numérotation des protons de ce dernier se fera à partir de l'hétéroatome en direction de la liaison sur le substrat principal.

### Spectres de RMN

### -exemple a, 5-Hydroxyindane-1,3-dione

7.78 (d, 1H, H-7, J H6-7 = 8.3 Hz)
7.26 (d ,1H, H-6, J H6-7 = 8.3 Hz)
7.11 (1s, 1H, H-4)
3.25 (s, 3H, CH2)

### -exemple b, 2-Bromo-5-hydroxyindane-1,3-dione

11,55 (1s, OH)
7.90 (dd, 1H, H-6, J H6-4 =2.93 Hz ; J H6-7 = 8.3 Hz)
7.39 (d, 1H, H-7, J H6-7 = 8.3 Hz)
7.21 (s, 1H, H-4)
5.50 (s, 1H, H2)

### -exemple c, 5-Hydroxyindane-1,2,3-trione, monohydrate

11.50 (1s, 1H, OH)
7.90 (d, 1H, H7, JH6-7 = 5.9 Hz)
7.40 (m, 2H, H4, H6)

### -exemple d, 5,6-Diméthoxyindane-1,2,3-trione, monohydrate

7.40 (1s, 2H, H-4, H-7)
4.03 (s, 6H, 2CH3O)

### -exemple e, 6-Hydroxy-5-méthoxyindane-1,2,3-trione, monohydrate

11.21 (1s, 1H, OH)
7.28 (m, 2H, H-4, H-7)
4.03 (s, 6H, 2CH3O)

### exemple f, 2-oximino-5-hydroxyindane-1,3-dione

9.16 (1s, 1H, OH)
7.83 (du, 1H, H6, JH6-7 = 9.0 Hz et JH4-6 = 3.0 Hz)
7.21 (m, 2H, H7, H4)

### -exemple 1, 2-Hydroxy-2-méthoxycarbonylhydrazinoindane-1,3-dione

8.22 (1s, 1H, NH)
8.01 (s ,4H, H-4, H-5,H-6, H-7)
6.77 (1s, 1H, NH)
5.86 (s, 1H, OH)
3.46 (s, 3H, CH3)

### -exemple 2, 2-Hydroxy-2-méthoxycarbonylhydrazino-5-méthoxyindane-1,3-dione

8.43 (1s, 1H, NH)
7.94 (dd, 1H, H-7, J H4-7 = 1.5 Hz, J H6-7 = 8.3 Hz)
7.53 (dd,1H, H-6, JH4-6 = 1.5 Hz, J H6-7 = 8.3 Hz)
7.39 (s, 1H, H-4)
6.78 (s, 1H, OH)
5.75 (1s, 1H, NH)
3.97 (s, 3H, CH3O)
3.47 (s, 3H, CH3OCO)

### -exemple 3, 2-Benzoylhydrazino-2-hydroxy-indane-1,3-dione

9.97 (1s, 1H, NH)
8.01 (s, 4H, H-4, H-5, H-6, H-7)
7.74 et 7.49 (2m ,5H, Ph)
7.32 (1s, 1H, OH)
6.33 (s, 1H, NH)

### -exemple 4, 2-Benzoylhydrazino-2-hydroxy-5-méthoxyindane-1,3-dione

9.91 (d, 1H, NH, J NH-NH = 3.9 Hz)
7.91 (d, 1H, H-7, J H 6-7 = 8.3 Hz)
7.75 (d, 1H, H-6, J H 6-7 = 8.3 Hz)
7.50 (m, 6H, H-4 et Ph)
7.09 (s, 1H, OH)
6.23 (d, 1H, NH, J NH-NH = 3.9 Hz)
3.97 (s, 3H, CH3O)

### 2-Hydroxy-2-(2-méthoxybenzoylhydrazino)-indane-1,3-dione

9.59 (d, 1H, NH, J NH-NH = 2.9 Hz)
8.03 (s, 4H, H-4, H-5, H-6, H-7)
7.74 (d, 1H, H-6', J H 5'-6' = 7.8 Hz)
7.50 (t, 1H, H-5', J H 5'-6' et 4'-5' = 7.8 Hz,)
7.15 (s, 1H, OH)
7.05 (m, 2H, H-3', H-5')
6.36 (d, 1H, NH, J NH-NH = 2.9 Hz)
3.91 (s, 3H, CH3O)

### 2-Hydroxy-2-(3-méthoxybenzoylhydrazino)-indane-1,3-dione

9.97 (1s, 1H, NH)
7.99 (s, 4H, H-4, H-5, H-6, H-7)
7.33 (m, 3H, H-2', H-3', H-4')
7.11 (1s, 2H, H-6', OH)
6.33 (1s, 1H, NH)
3.77 (s, 3H, OCH3)

### 2-Hydroxy-2-(3-méthoxybenzoylhydrazino)-5-méthoxy indane-1,3-dione

9.91 (1s, 1H, NH)
7.93 (d, 1H, H-7, J H6-7 = 8.3 Hz)
7.53 (d, 1H, H-6, J H6-7 = 8.3 Hz)
7.35 (m, 5H, H-4 et Ph)
7.10 (s,1H, OH)
6.22 (1s, 1H, NH)
3.97 (s, 3H, CH3O)
3.78 (s, 3H, CH3O, Ph)

### 2-Hydroxy-2-(4-méthoxybenzoylhydrazino)- indane-1,3-dione

9.83 (1s, 1H, NH, )
7.75 (d, 2H, H-2', H-6', J H5'-6' et 2'-3' = 8.3 Hz)
8.00 (s, 4H, H-4, H-5, H-6, H-7)
7.17 (s,1H, OH)
6.98 (d, 2H, H-3', H-5', J H5'-6' et 2'-3' = 8.3 Hz)
6.29 (1s, 1H, NH)
3.80 (s, 3H, CH3O)

### -exemple 6, 2-Hydroxy-2-(2-méthylbenzoylhydrazino)- indane-1,3-dione

9.69 (d, 1H, NH, J NH-NH = 3.9 Hz)
8.01 (s, 4H, H-4, H-5, H-6, H-7)
7.25 (m, 4H, H-3',H-4', H-5', H-6')
7.06 (s, 1H, OH)
6.32 (d, 1H, NH, J NH-NH = 3.9 Hz)
2.26 (s, 3H, CH3)

### -exemple 7, 2-Hydroxy-2-(3-méthylbenzoylhydrazino)- indane-1,3-dione

9.90 (1s, 1H, NH)
8.00 (s, 4H, H-4, H-5, H-6, H-7)
7.55 et 7.30 (2m, 4H, H-2',H-4', H-5', H-6')
7.08 (s, 1H, OH)
6.30 (1s, 1H, NH)
2.35 (s, 3H, CH3)

### -exemple 8, 2-Hydroxy-2-(4-méthylbenzoylhydrazino)- indane-1,3-dione

9.89 (d, 1H, NH, J NH-NH = 3.9 Hz)
8.00 (s, 4H, H-4, H-5, H-6, H-7)
7.66 (d, 2H, H-2', H-6', J H2'-3' et 5'-6' = 8.3 Hz)
7.25 (d, 2H, H-3', H-5', J H2'-3' et 5'-6' = 8.3 Hz)
7.12 (s, 1H, OH)
6.30 (d, 1H, NH, J NH-NH = 3.9 Hz)
2.35 (s, 3H, CH3)

### -exemple 9, 2-Hydroxy-2-(2-hydroxybenzoylhydrazino)- indane-1,3-dione

11.70 (s, 1H, PhOH)
10.07 (1s, 1H, NH)
8.00 (s, 4H, H-4, H-5, H-6, H-7)
7.84 (d, 1H, H-6', J H5'-6' = 7.8 Hz)
7.40 (m, 1H, H-5')
7.05 (s, 1H, OH)
6.90 (m, 2H, H-3', H-4')
6.35 (1s, 1H, NH)

### -exemple 10, 2-Hydroxy-2-(3-hydroxybenzoylhydrazino)- indane-1,3-dione

9.83 (d, 1H, NH, J NH-NH =3.9 Hz)
9.70 (s, 1H, PhOH)
8.00 (s, 4H, H-4, H-5, H-6, H-7)
7.15 (m, 5H, H-2',H-4', H-5', H-6', OH)
6.30 (d, 1H, NH, J NH-NH = 3.9 Hz)

### -exemple 11, 2-(3,4-Diméthoxybenzoylhydrazino)-2-hydroxyindane -1,3-dione

9.85 (1s, 1H, NH)
8.00 (s, 4H, H-4, H-5, H-6, H-7)
7.35 (m, 4H, H-2', H-5', H-6', OH)
6.30 (1s, 1H, NH)
3.78 et 3.79 (2s, 6H, CH3O)

### -exemple 12, 2-Hydroxy-2-(2-hydroxy-3-méthylbenzoylhydrazino) indane-1,3-dione

12.39 (s, 1H, PhOH)
10.31 (1s, 1H, NH)
8.02 (s, 4H, H-4, H-5, H-6, H-7)
7.74 (d, 1H, H-6', J H5'-6' = 7.8 Hz)
7.30 (d, 1H, H-4', J H4'-5' = 7.8 Hz)
6.97 (d, 1H, OH)
6.79 (t, 1H, H-5', J H5'-6' et 4'-5' = 7.8 Hz)
6.41 (1s, 1H, NH)
2.15 (s, 3H, CH3)

### -exemple 13, 2-Hydroxy-2-(4-hydroxy-3-méthoxybenzoylhydrazino) indane-1,3-dione

9.9 (1s, 1H, NH)
9.82 (s, 1H, PhOH)
8.00 (s, 4H, H-4, H-5, H-6, H-7)
7.38 (m, 3H, H-2', H-6', OH)
6.80 (d, 1H, H-5', J H5'-6' = 8.3 Hz)
6.34 (1s, 1H, NH)
3.81 (s, 3H, CH3O)

### -exemple 14, 2-Hydroxy-2-(5-phényl-2-(N-pyrrolyl)thiophène-3-carbonylhydrazino)-indane-1,3-dione

10.05 (s, 1H, OH)
9.9 (1s, 1H, NH)
8.00 (s, 4H, H-4, H-5, H-6, H-7)
7.66 et 7.38 (m, 7H, 2H pyrrole, 5H phenyl)
7.05 (s, 3H, OH, 2H pyrrole)
6.36 (1s, 1H, NH)
6.25 (s, 1H, thiophene)

### 2-Hydroxy-2-nicotinoylhydrazino-indane-1,3-dione

10.23 (1s, 1H, NH)
8.94 (s, 1H, H-2')
8.75 (m, 1H, H-6')
8.73 (m, 1H, H-4')
8.05 (s, 4H, H-4, H-5, H-6, H-7)
7.54 (m, 1H, H-5')
7.09 (s, 1H, OH)
6.43 (1s, 1H, NH)

### 2-Hydroxy-2-isonicotinoylhydrazino-indane-1,3-dione

10.27 (1s, 1H, NH)
8.02 (m, 2H, H-2', H-6')
8.02 (s, 4H, H-4, H-5, H-6, H-7)
7.69 (m, 2H, H-3', H-5')
7.04 (s, 1H, OH)
6.42 (1s, 1H, NH)

### -exemple 15, 2-Hydroxy-2-oxamoylhydrazino-indane-1,3-dione

9.53 (1s, 1H, NH)
8.11 (s, 1H, NH)
8.02 (s, 4H, H-4, H-5, H-6, H-7)
7.86 (s, 1H, OH)
7.17 (s, 1H, OH)
6.20 (1s, 1H, NH)

### 2-Hydroxy-2-isonicotinoylhydrazino-5-méthoxy-indane-1,3-dione

10.22 (d, 1H, NH, J NH-NH = 3.9 Hz)
8.73 (d, 2H, H-3', H-5', J H2'-3' et 5'-6'= 5.8 Hz)
7.94 (d,1H, H-7, , J H6-7 = 8.8 Hz)
7.69 (d,2H, H-2', H-6', J H2'-3' et 5'-6'= 5.8 Hz)
7.45 (dd, 1H, H-6, , J H4-6 = 2.4 et 6-7= 8.8Hz)
7.41 (d,1H, H-4, J H4-6 = 2.4Hz)
7.04 (s, 1H, OH)
6.30 (d, 1H, NH, J NH-NH = 3.9 Hz)
3.38 (s,3H, CH3)

### -exemple 16, 2-Hydroxy-2-(indole-3-acétylhydrazino)-indane-1,3-dione

10.84 (s, 1H, NH)
9.52 (d, 1H, NH, J NH-NH = 3.9 Hz)
7.95 (s, 4H, H-4, H-5, H-6, H-7)
7.50 (d,1H, H-4', J H4'-5' = 7.8 Hz)
7.30 (d, 1H, H-5', J H4'-5' = 7.8 Hz)
7.14 (s, 1H, OH)
6,94 à 7.04 (m, 3H, H-2', H-6', H-7')
6.10 (d, 1H, NH, J NH-NH = 3.9 Hz)
3.45 (s, 2H, CH2)

### 2-Benzamido-2-hydroxyindane-1,3-dione

9.85 (s, 1H, NH)
8.04 (s, 4H, H-4, H-5, H-6, H-7)
7.91 (m, 3H, OH, H-2', H-6')
7.49 (m, 3H, H-3', H-4', H-5')

### -exemple 17, 2-Chloracétamido-2-hydroxyindane-1,3-dione

9.62 (s, 1H, NH)
8.04 (s, 4H, H-4, H-5, H-6, H-7)
7.97 (s, 1H, OH)
4.12 (s, 2H, CH2)

### 2-Benzamido-2-chloroindane-1,3-dione

9.85 (1s, 1H, NH)
8.04 (s, 4H, H-4, H-5, H-6, H-7)
7.90 (m, 2H, H-2', H-6')
7.46 (m, 3H, H-3', H-4', H-5')

### -exemple 18, 2-(4-Aminobenzoylhydrazino)-2-hydroxy-indane-1,3-dione

9.53 (1s, 1H, NH)
7.99 (s, 4H, H-4, H-5, H-6, H-7)
7.50 (d, 2H, H-2', H-6', J H2'-3' et 5'-6' = 8.3 Hz)
7.40 (s, 1H, OH)
6.53 (d, 2H, H-3', H-5', J H2'-3' et 5'-6' = 8.3 Hz)
6.25 (1s, 1H, NH)
5.72 (m, 2H, NH2)

### -exemple 19, 2-(4-Aminoéthylmorpholino)-2-benzamidoindane-1,3-dione

9.62 (s, 1H, NH)
7.99 (s, 4H, H-4, H-5, H-6, H-7)
7.81 (d, 2H, H-2', H-6', J H2'-3' et 5'-6' = 7.8 Hz)
7.50 (m, 3H, H-3', H-4', H-5')
3.50 (m, 4H, 2CH2)
3.22 (s, 1H, NH)
2.28 et 2.37(2m, 8H, 4CH2, morpholino)

### -exemple 20, 2-Benzamido-2-[1-(2-pyridyl)-pipérazino]indane-1,3-dione

9.46 (s, 1H, NH)
8.08 (m, 1H, H6 de pyridine)
8.00 (s, 4H, H-4, H-5, H-6, H-7)
7.87 (d, 2H, H-2', H-6', J H2'-3' et H5'-6'= 6.8 Hz)
7.50 (m, 4H, H-3', H-4', H-5', H5 de pyridine)
6.75 (d, 1H, H-3 de pyridine, J H3-H4' = 8.31 Hz)
6.60 (m, 1H, H-4 de pyridine)
3.38 (m, 8H, 4CH2, pipérazino)

### -exemple 21, 2-Benzamido-2-[1-(4-fluorophényl)-pipérazino]indane-1,3-dione

12.00(s, 1H, NH)
8.00 (s, 4H, H-4, H-5, H-6, H-7)
7.92 (d, 2H, H-2', H-6', J H2'-3' et H5'-6'= 7.3 Hz)
7.51 (m, 4H, H-3', H-4', H-5')
6.97.(m, 2H, H-3 et H-5 du phényl fluoré)
6.85 (m, 2H, H 2 et H-6 du phényl fluoré)
3.00 (m, 8H, 4CH2, pipérazino)

### -exemple 22, 2-Méthoxycarbonylhydrazono-indane-1,3-dione

12.19 (1s, 1H, NH)
7.99 (s, 4H, H-4, H-5, H-6, H-7)
3.86 (s, 3H, CH3)

### -exemple 23, 2-Methoxycarbonylhydrazono-5-méthoxyindane-1,3-dione

12.19 (1s, 1H, NH)
7.91 (dd, 1H, H-6, J H6-7 = 8.3 Hz, J H6-4 = 1.46Hz)
7.52 (d, 1H, H-7, J H7-6 = 8.3Hz)
7.34 (s, 1H, H-4)
3.97 (s, 3H, CH3)
3.47 (s, 3H, CH3)

### -exemple 24, 2-Benzoylhydrazonoindane-1,3-dione

13.60 (1s,1H, NH)
8.03 (s, 4H, H-4, H-5, H-6, H-7)
7.94 (d, 2H, H-2', H-6', J H2'-3' et H5'-6' = 7.8Hz))
7.66 (m, 3H, H-3', H-4', H-5')

### -exemple 25, 2-Benzoylhydrazono-5-méthoxyindane-1,3-dione

13.55 (s,1H, NH)
7.94 (m, 3H, H-4, H-6, H-7)
7.74 (m, 2H, H-2', H-6')
7.66 (m, 3H, H-3', H-4', H-5')
3.98 (s, 3H, OCH3)

### 2-(2-Méthoxybenzoylhydrazono)-indane-1,3-dione

14.26 (s, 1H, NH)
8.12 (d, 1H, H-3', J H3'-4' = 7.3Hz)
8.00 (s ,4H, H-4, H-5,H-6, H-7)
7.70 (m, 1H, H-4')
7.36 (d, 1H, H-6', J H5'-6' = 8.3 Hz)
7.18 (m, 1H, H-5')
4.21 (s, 3H, OCH3)

### 2-(3-Méthoxybenzoylhydrazono)-indane-1,3-dione

12.3 (s,1H, NH)
8.00 (s ,4H, H-4, H-5,H-6, H-7)
7.50 (m, 2H, H-2', H-4')
7.27 (m, 2H, H-5', H-6')
3.88 (s, 3H, OCH3)

### 2-(3-Méthoxybenzoylhydrazono)-5-méthoxyindan-1,3-dione

12.3 (1s, 1H, NH)
7.99 (s, 4H, H-4, H-5, H-6, H-7)
7.33 (m, 3H, H-2', H-3', H-4')
7.11 (s, 1H, H-6')
3.77 (s, 3H, OCH3)

### 2-(4-Méthoxybenzoylhydrazono)-indane-1,3-dione

13.58 (s,1H, NH)
8.00 (s ,4H, H-4, H-5,H-6, H-7)
7.92 (d, 2H, H-2', H-6'J H2'-3' et H5'-6' = 8.7Hz)
7.18 (d, 2H, H-3', H-5'J H2'-3' et H5'-6' = 8.7Hz)
3.88 (s, 3H, OCH3)

### -exemple 26, 2-(2-Méthylbenzoylhydrazono)-indane-1,3-dione

13.04 (s,1H, NH)
8.01 (s ,4H, H-4, H-5,H-6, H-7) 7.57 (m, 2H, H-4', H-5')
7.43 (m, 2H, H-3', H-6')
3.55 (s, 3H, CH3)

### -exemple 27, 2-(3-Méthylbenzoylhydrazono)-indane-1,3-dione

13.58 (s,1H, NH)
8.01 (s ,4H, H-4, H-5,H-6, H-7)
7.72 (m, 2H, H-2', H-6')
7.53 (m, 2H, H-4', H-5')
3.33 (s, 3H, CH3)

### -exemple 28, 2-(4-Méthylbenzoylhydrazono)-indane-1,3-dione

13.59 (s,1H, NH)
8.02 (s ,4H, H-4, H-5,H-6, H-7)
7.84 (d, 2H, H-2', H-6', J H2'-3' et H5'-6' = 7.8Hz)
7.44 (d, 2H, H-3', H-5', J H2'-3' et H5'-6' = 7.8Hz)
3.34 (s, 3H, CH3)

### -exemple 29, 2-(2-Hydroxybenzoylhydrazono)-indane-1,3-dione

14.48 (s,1H, NH)
8.04 (s ,4H, H-4, H-5,H-6, H-7)
7.50 (m, 1H, H-3')
7.05 (m, 3H, H-4', H-5', H-6')

### -exemple 30, 2-(3-Hydroxybenzoylhydrazono)-indane-1,3-dione

13.58 (s,1H, NH)
10.17 (s,1H, OH)
8.04 (s ,4H, H-4, H-5,H-6, H-7)
7.47 (m, 3H, H-2', H-5', H-6')
7.34 (m, 1H, H-4')

### -exemple 31, 2-(4-Hydroxybenzoylhydrazono)-indane-1,3-dione

13.58 (s,1H, NH)
10.62 (s,1H, OH)
8.01 (s ,4H, H-4, H-5,H-6, H-7)
7.83 (d, 2H, H-2', H-6', J H2'-3' et H5'-6' = 8.3Hz)
7.00 (d, 2H, H-3', H-5', J H2'-3' et H5'-6' = 8.3Hz)

### -exemple 32, 2-(3,4-Dihydroxybenzoylhydrazono)-indane-1,3-dione

9.44 (s,1H, NH)
8.01 (s ,4H, H-4, H-5,H-6, H-7)
7.36 (s, 1H, H-2')
7.25 (d, 1H, H-5', J H5'-6' = 8.3Hz)
6.74 (d, 1H, H-6', J H5'-6' = 8.3Hz)

### -exemple 33, 2-(5-Phényl-2-(N-pyrrolyl)thiophène-3-carbonylhydrazono)-indane-1,3-dione

12.78 (s,1H, NH)
7.98 (s ,4H, H-4, H-5,H-6, H-7)
7.70 (m, 3H, H2', H6', H thiophène, )
7.44 (m, 3H, H3', H4', H5')
7.12 (s, 2H, H pyrrole)
6.25 (s, 2H, H-N pyrrole)

### 2-Nicotinoylhydrazonoindan-1,3-dione

13.41 (s,1H, NH)
9.06 (s, 1H, H-2')
8.85 (d, 1H, H-6', J H5'-6' = 4.4 Hz)
8.27 (d, 1H, H-4', J H4'-5' = 6.8 Hz)
8.01 (s ,4H, H-4, H-5,H-6, H-7)
7.67 (m, 1H, H-5')

### -exemple 34, 2-Oxamoylhydrazonoindane-1,3-dione

13.80 (s,1H, NH)
8.70 (s, 1H, NH, dans le DMSO il est en équilibre céto-énolique)
8.32 (s, 1H, OH, dans le DMSO il est en équilibre céto-énolique)
8.02 (s ,4H, H-4, H-5,H-6, H-7)

### -exemple 35, 2-(3-Hydroxybenzoylhydrazono)-5-méthoxyindane-1,3-dione

13.62 (s,1H, OH)
10.07 (s, 1H, NH)
8.00 (d, 1H, H-6, J H6-7 = 8.3 Hz)
7.95 (m, 6H, H-4, H-5, H-7, H-2', H-4', H5')
7.32 (d, 1H, H-6', J H5'-6' = 8.2 Hz)
4.01 (s, 3H, CH3)

### -exemple 36, 2-(3,4-Diméthoxybenzoylhydrazono)-indane-1,3-dione

12.3 (1s, 1H, NH)
8.00 (s, 4H, H-4, H-5, H-6, H-7)
7.35 (m, 3H, H-2', H-5', H-6')
3.78 et 3.79 (2s, 6H, CH3O)

### -exemple 37, 2-(2-Hydroxy-3-méthylbenzoylhydrazono)-indane-1,3-dione

14.51 (s,1H, NH)
11.08 (s, 1H, OH)
8.08 (s, 4H, H-4, H-5, H-6, H-7)
7.85 (d, 1H, H-6', J H5'-6' = 7.8 Hz)
7.42 (d, 1H, H-4', J H4'-5' = 7.3 Hz)
6.95 (m, 1H, H-5')
2.30 (s, 3H, CH3)

### -exemple 38, 2-(4-Hydroxy-3-méthoxybenzoylhydrazono)-indane-1,3-dione

14.51 (s,1H, NH)
11.08 (s, 1H, OH)
8.01 (m, 4H, H-4, H-5, H-6, H-7)
7.50 (s, 1H, H-2')
7.43 (d, 1H, H-6', J H5'-6' = 8.3 Hz)
7.00 (d, 1H, H-5', J H5'-6' = 8.3 Hz)
3.88 (s, 3H, CH3)

### Isonicotinoylhydrazino-indane-1,3-dione

13.47 (1s, 1H, NH)
8.90 (d, 2H, H-3', H-5', J H2'-3' et JH5'-6'= 6.35 Hz)
8.03 (s, 4H, H-4, H-5, H-6, H-7)
7.81 (m, 2H, H-2', H-6', J H2'-3' et JH5'-6'= 6.35 Hz)

### 2-Isonicotinoylhydrazono-5-méthoxyindane-1,3-dione

13.47 (s, 1H, NH)
8.73 (d, 2H, H-3', H-5', J H2'-3' et 5'-6'= 5.8 Hz)
7.94 (d,1H, H-7, ,J H6-7 = 8.8 Hz)
7.69 (d,2H, H-2', H-6', J H2'-3' et 5'-6'= 5.8 Hz)
7.45 (dd, 1H, H-6, , J H4-6 = 2.4 et 6-7= 8.8Hz)
7.41 (d, 1H, H-4, J H4-6 = 2.4Hz)
3.38 (s,3H, CH3)

### -exemple 39, 2-(2-Thénoylhydrazono)-indane-1,3-dione

13.09 (1s, 1H, NH)
8.13 (d, 1H, H-3', J H3'-4' = 4.9 Hz)
8.05 (m, 1H, H-5')
8.02 (s, 4H, H-4, H-5, H-6, H-7)
7.34 (t, 1H, H-4', J H3'-4' et J H4'-5' = 4.9 Hz)

### -exemple 40, 2-(2-Furoylhydrazono)-indane-1,3-dione

13.49 (1s, 1H, NH)
8.13 (1s, 1H, H-3')
8.01 (s, 4H, H-4, H-5, H-6, H-7)
7.56 (m, 1H, H-5')
6.85 (m, 1H, H-4')

### -exemple 41, 2-(4-Aminobenzoylhydrazono)-indane-1,3-dione

13.61 (1s, 1H, NH)
8.00 (s, 4H, H-4, H-5, H-6, H-7)
7.67 (d, 2H, H-2', H-6', J H2'-3' et J H5'-6' = 8.3 Hz)
6.71 (d, 2H, H-3', H-5', J H2'-3' et J H5'-6' = 8.3 Hz)
6.30 (1s, 2H, NH2)

### -exemple 42, 2-(4-Trifluoroacétylaminobenzoylhydrazono)-indane-1,3-dione

13.58 (1s, 1H, NH)
11.68 (1s, 1H, NH)
8.00 (s, 4H, H-4, H-5, H-6, H-7)
7.96 (s, 4H, Phe)

### -exemple 43, 2-(4-Acétylaminobenzoylhydrazono)-indane-1,3-dione

13.56 (1s, 1H, NH)
10.38 (1s, 1H, NH)
8.00 (s, 4H, H-4, H-5, H-6, H-7)
7.83 (m, 4H, Phe)
2.08 (s, 3H, CH3)

### 2-Phénylhydrazonoindane-1,3-dione

13.16 (1s, 1H, NH)
7.89 (s, 4H, H-4, H-5, H-6, H-7)
7.63 (d, 2H, H-2', H-6', J H2'-3' et J H5'-6' = 8.1 Hz)
7.46 (t, 2H, H-3', H-5', J H2'-3' et J H5'-6' = 8.1 Hz)
7.24 (m, 1H, H-4')

### -exemple 50, 2-(2,4-Dichlorophénylhydrazono)-indane-1,3-dione

12.64 (1s, 1H, NH)
7.96 (s, 4H H-4, H-5, H-6, H-7)
7.66 (s, 1H, H-3')
7.44 (d, 2H, H-5', H-6', J H5'-6' = 8.8 Hz)

### -exemple 51, 2-(3-Chloro-4-méthylphénylhydrazono)-indane-1,3-dione

13.01 (1s, 1H, NH)
7.87 (s, 4H, H-4, H-5, H-6, H-7)
7.65 (s, 1H, H-2')
7.45 (m, 2H, H-5', H-6')
2.27 (s, 3H, CH3)

### 2-(3-Fluorophénylhydrazono)-indane-1,3-dione

13.05 (1s, 1H, NH)
7.90 (s, 4H, H-4, H-5, H-6, H-7)
7.47 (m, 3H, H-2', H-4', H-6')
7.01 (m, 1H, H-5')

### 2-(3-Fluorophénylhydrazono)-5-méthoxyindane-1,3-dione

12.94 (1s, 1H, NH)
7.89 (d, 1H, H7, J H6-7 = 8.3 Hz)
7.97 (m, 5H, H4, H-6, H-2', H-4', H-6')
7.31 (m, 1H, H-5')
3.95 (s, 3H, OCH3)

### 2-(4-Fluorophénylhydrazono)-indane-1,3-dione

13.56 (1s, 1H, NH)
7.88 (s, 4H, H-4, H-5, H-6, H-7)
7.70 (m, 2H, H-3', H-5')
7.63 (m, 2H, H-2', H-6')

### 2-(3-Méthylphénylhydrazono)-indane-1,3-dione

13.11 (1s, 1H, NH)
7.85 (s, 4H, H-4, H-5, H-6, H-7)
7.35 (m, 3H, H-2', H-5', H-6')
7.00 (d, 1H, H-4', J H4'-5' = 6.8 Hz)
3.38 (s, 3H, CH3)

### 2-(4-Méthoxyphenylhydrazono)-indane-1,3-dione

13.28 (1s, 1H, NH)
7.85 (s, 4H, H-4, H-5, H-6, H-7)
7.58 (d, 2H, H-2', H-6',J H2'-3' et J H5'-6' = 8.8 Hz)
7.02 (d, 2H, H-3', H-5', J H2'-3' et J H5'-6' = 8.3 Hz)
3.77 (s, 3H, OCH3)

### 2-(4-Méthoxyphénylhydrazono)-5-methoxyindane-1,3-dione

13.12 (1s, 1H, NH)
7.77 (d, 1H, H-7, J H6-H7 = 8.3 Hz)
7.53 (d, 2H, H-2', H-6', J H2'-3' et J H5'-6' = 8.8 Hz)
7.34 (m, 2H, H-4, H-6)
7.00 (d, 2H, H-3', H-5', J H2'-3' et J H5'-6' = 8.8 Hz)
3.77 (s, 3H, OCH3)

### -exemple 52, 2-(4-méthylsulfonylphénylhydrazono)-indane-1,3-dione

13.73 (1s, 1H, NH)
7.80 (m 8H, H-4, H-5, H-6, H-7, H-2', H-3', H-5', H-6')
3.22 (s, 3H, OCH3)

### -exemple 53, 5-Hydroxy-2-(4-méthylsulfonylphénylhydrazono)-indane-1,3-dione

12.93 (1s, 1H, NH)
11.16 (1s, 1H, OH)
7.78 (d, 2H,, H-3', H-5', J H2'-3' et J H5'-6' = 7.8 Hz)
7.20 (m, 3H, H-6, H-2', H-6')
7.21 (d, 1H, H-7, J H6-7 = 8.3 Hz)
7.15 (s, 1H, H-4)
3.23 (s, 3H, OCH3)

### -exemple 54, 2-(4-N-Méthylméthanesulfonamidophénylhydrazono)-indane-1,3-dione

13.17 (1s, 1H, NH)
7.88 (s, 4H, H-4, H-5, H-6, H-7)
7.63 (d, 2H, H-2', H-6', J H2'-3' et J H5'-6' = 8.8 Hz)
7.42 (d, 2H, H-3', H-5', J H2'-3' et J H5'-6' = 8.8 Hz)
6.96 (d, 1H, NH, J NH-CH3 = 4.4 Hz)
4.34 (s, 2H, CH2)
2.56 (d, 3H, CH3, J NH-CH3 = 4.4 Hz)

### -exemple 55, 5-Méthoxy-2-(4-N-méthylméthanesulfonamidophénylhydrazono)-indane-1,3-dione

13.04 (1s, 1H, NH)
7.82 (d, 1H, H-7J, J H6-7 = 8.3 Hz)
7.58 (d, 2H, H-2', H-6', J H2'-3' et J H5'-6' = 8.3 Hz)
7.42 (d, 2H, H-3', H-5', J H2'-3' et J H5'-6' = 8.3 Hz)
7.38 (m, 2H, H-4, H-6)
6.95 (d, 1H, NH, J NH-CH3 = 4.9 Hz)
4.33 (s, 2H, CH2)
3.38 (s, 3H, CH3O)
2.58 (d, 3H, CH3, J NH-CH3 = 4.9 Hz)

### -exemple 56, 2-(2-Hydroxyphényl)-iminoindane-1,3-dione

8.00 (s, 4H, H-4, H-5, H-6, H-7)
7.85 (m, 4H, H-3', H-4', H-5', H-6')

### -exemple 62, 2-Oximino-1-(4-phénylthiosemicarbazono)-3-indanone

9.40 (1s, 1H, OH)
9.30 (1s, 1H, NH)
8.36 (d, 1H, H-7, J H6-7 = 7.3 Hz)
7.73 (m, 5H, H-4, H-5, H-6, H-2', H-6')
7.31 (t, 2H, H-3', H-5', J H2'-3' et JH5'-6' = 7.3 Hz)
7.06 (t, 1H, H-4', J H3'-4' et JH4'-5' = 7.3 Hz)

### -exemple 63, 1,3-Dioximino-2-(4-phénylthiosemicarbazono)-3-indanone

13.06 (1s, 1H, NH)
12.59 (1s, 1H, OH)
11.63 (1s, 1H, OH)
9.19 (1s, 1H, NH)
8.56 (d, 1H, H-7, H-4, J H6-7 et J H4-5 = 7.3 Hz)
7.64 (m, 4H, H-5, H-6, H-2', H-6')
7.33 (t, 2H, H-3', H-5', J H2'-3' et JH5'-6' = 7.3 Hz)
7.03 (t, 1H, H-4', J H3'-4' et JH4'-5' = 7.3 Hz)

La présente invention s'étend par ailleurs à l'utilisation en thérapeutique humaine et vétérinaire d'une part des composés de formule : 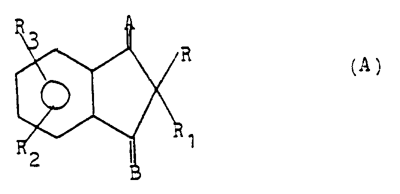
dans laquelle R₂ et R₃ représentent indépendamment l'un de l'autre H, alkoxy en C₁-C₄ ou OH ; et le couple (A, B) prend la valeur :
- (oxygène, oxygène), auquel cas l'un parmi R et R₁ représente OH, halogène, (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène et l'autre représente un groupe choisi parmi les suivants :
   . NHCOR₄ où R₄ = phényle ; alkyle en C₁-C₄ ; ou alkyle en C₁-C₄ substitué par un halogène,
   . NHNHXOR₅ où R₅ = alkoxy en C₁-C₄ ; phényle, phényle substitué par un ou deux groupes choisis parmi les suivants : amino, alkoxy en C₁-C₄, alkyle en C₁-C₄, OH ; thiényle ; furyle ; pyridyle ; indolyl-2_méthyle ; indolyl-3-méthyle ; ou 5-phényl-2-(N-pyrrolyl)thiényle,
   . NHNHCOCONH₂, R et R₁ pouvant par ailleurs former ensemble un groupe :
   . =NNHCOR'₅ où R'₅ = R₅, trifluoroacétylaminophényle, acétylaminophényle, pyrazinyle ou pyrrolyle,
   . =NNHCOCONH₂,
   . =NNHR₆ où R₆ = phényle ; phényle substitué par un ou deux groupes choisis parmi alkyle en C₁-C₄, halogène et alkoxy en C₁-C₄ ; méthylsulfonylphényle ; et N-méthylméthanesulfonamidophényle,
   . =N-R₇ où R₇ = phényle ou phényle substitué par un groupe OH,
   . =N-O-CO-R₈ où R₈ = phényle ou phényle substitué par un atome d'halogène, ou
   . =C(CH₃)-NH-NH-CO-R₉ où R₉ = phényle, phénylamino, phényle substitué par un atome d'halogène ou un groupe OH, ou phénylamino substitué par un atome d'halogène ou un groupe OH ; ou
- (NNHCXNHR₁₀, oxygène) où X représente oxygène ou soufre et R₁₀ = H, phényle ou phényle substitué par un ou deux groupes choisis parmi OH, CF₃, alkyle en C₁-C₄, alkoxy en C₁-C₄, halogène, méthylènedioxy, acétoxy et hydroxyéthyle, auquel cas R et R₁ forment ensemble un groupe =N-OH,
   à l'exclusion des composés pour lesquels R et R₁ forment ensemble un groupe =N-NH-CO-R'₅ où R'₅ = pyridyle ; et
   d'autre part des sels d'addition d'acide minéral ou organique, pharmaceutiquement acceptables, des composés salifiables parmi ceux de formule (A) ci-dessus.

Les composés de formule (A) et leurs sels éventuels, non englobés dans la formule (I), sons connus et peuvent être préparés selon les schémas ① à ⑤ ci-dessus en mettant en oeuvre les produits de départ appropriés.

L'étude des composés de formule (A) et de leurs sels éventuels a montré qu'ils possèdent diverses propriétés pharmacologiques. Ainsi, ils sont veinotoniques et n'affectent pas dans la plupart des cas le système artériel ; par ailleurs, ils augmentent la résistance capillaire, diminuent l'hyperperméabilité vasculaire induite par certains agents inflammatoires et présentent des propriétés antilipoperoxydantes, antiradicalaires et antiinflammatoires, ainsi qu'une activité dans le choc septique.

Ces propriétés sont démontrées chez les mammifères tels que les rats, cobayes et lapins, dans des conditions in vitro (vaisseaux ou réseaux vasculaires isolés) et in vivo.

Pour l'étude in vitro, les composés sont solubilisés en solution aqueuse pure ou contenant du DMSO ou de l'alcool.

Pour l'étude in vivo, ils sont administrés par voie intra-veineuse sous forme de solution aqueuse pure, par voie intrapéritonéale sous forme de solution aqueuse contenant ou non du DMSO ou par voie orale en solution ou en suspension dans la carboxyméthylcellulose ou dans une solution aqueuse composée contenant du Tween^{R} et dans certains cas du DMSO.

### Modèles d'études pharmacologiques

L'effet contractile est mesuré in vitro :
- par la force de contraction développée par des anneaux vasculaires quiescents ou stimulés (soit électriquement soit par des agents physiologiques) et maintenus dans des conditions isométriques,
- par la pression développée par des réseaux vasculaires perfusés à débit constant.

In vivo, les pressions artérielles et veineuses sont mesurées dans des conditions normales et après arrêt cardiaque. Lors de l'arrêt cardiaque, le tonus veineux est calculé à partir des pressions veineuses et artérielles mesurées à l'équilibre et corrigées en fonction des différences relatives de compliance entre ces deux réseaux (Samar et Coleman, Am. J. Physiol., 1978, 234:H94-100 ; Yamamoto et col., Am. J. Physiol., 1980, 238:H823-828).

L'augmentation de la résistance capillaire est appréciée par la modification de l'index pétéchial (pression négative induisant l'extravasation d'érythrocytes), mesuré par une méthode dérivée de l'angiosterromètre de Parrot.

La perméabilité vasculaire est étudiée in vivo et in vitro par la mesure de l'extravasation d'albumine ou de colorant liant l'albumine (Bleu Evans). In vivo, l'hyperperméabilité est induite par l'injection d'une solution d'histamine, de bradykinine ou de zymosan. Les modèles in vitro permettent de réaliser des hyperpressions (sur un territoire vasculaire isolé) et/ou des réactions vasculaires inflammatoires.

L'activité antiinflammatoire est démontrée par la mesure de l'inhibition de l'oedème et de la migration leucocytaire après induction d'une pleurésie chez le rat par injection de carragénine dans la cavité pleurale (Almeida et col., J. Pharmacol. Exp. Therap., 1980, 214:74).

L'effet "piégeur de radicaux libres" global est étudié in vitro par un modèle utilisant le 1,1-diphényl-2-picrylhydrazyl (DPPH) comme radical libre stable, méthode dérivée de celle décrite par Lamaison et col., Plantes Médic. et phytothérapie, XXII, 1988, 231-234.

L'activité antioxydante est étudiée in vitro par un modèle de peroxydation lipidique basée sur la peroxydation d'une émulsion d'acide linoléique par le fer, méthode modifiée par rapport à celle décrite par Sutherland et col., Arch. Biochem. Biophys., 1982, 214,1-11.

L'activité dans le choc septique est étudiée chez le rat après induction par une endotoxine lipopolysaccharidique (15 mg/kg), méthode voisine de celle décrite par Terashita et col., Eur. J. Pharmacol. 109, 257-261, 1985.

### Exemples d'effets pharmacologiques

Les composés de formule (A) et leurs sels éventuels augmentent la contraction des veines saphènes animales produite par la noradrénaline et la dépolarisation (solution hyperpotassique) sans affecter, dans la majorité des cas, les réponses contractiles artérielles. Ainsi, la 2-hydroxy-2-(2-méthoxybenzoylhydrazino)-indane-1,3-dione et le composé de l'exemple 22 (10 nM à 30 µm) augmentent de plus de 50 % (DE₅₀ ± 0,3 microM) les contractions des veines saphènes de lapins produites par le KCl (40 mM).

Les composés des exemples 3 et 12 et la 2- (3-méthylphénylhydrazono)-indane-1,3-dione augmentent à leur concentration maximale de 30 à 200 % la concentration des veines saphènes de lapin en réponse à la noradrénaline 0,3 micromolaire.

Les composés objet des exemples 3, 6 et 11 augmentent de plus de 20 % le tonus veineux de base du rat sans affecter la pression artérielle à des doses de 1 à 3 picomoles en administration i.v.

A titre illustratif, les composés des exemples 17, 22 et 54 et la 2-isonicotinoylhydrazonoindane-1,3-dione augmentent la résistance capillaire de base de 10 à 100 %, lorsque celle-ci est mesurée une heure à deux heures après administration de 5-20 mg/kg/i.p. et jusqu'à quatre à six heures après administration orale de 5-20 mg/kg chez le rat.

Les composés des exemples 1, 3 et 11 et la 2-hydroxy-2-(3-méthoxybenzoylhydrazino)-indane-1,3-dione diminuent la perméabilité vasculaire de 10 à 50 %, lorsque celle-ci est mesurée une à deux heures après administration de 5-20 mg/kg/i.p. et deux à quatre heures après administration orale de. 5-20 mg/kg chez le rat.

Le composé de l' exemple 9 et la 2-isonicotinoylhydrazonoindane-1,3-dione, administrés deux fois en i.p. à la dose de 20 mg/kg inhibent l'oedème et la migration leucocytaire dans la cavité pleurale, 6 heures après injection de carragénine dans le modèle de la pleurésie chez le rat.

Les composés des exemples 9 et 56 et la 2- (3-fluorophénylhydrazono)-5-méthoxyindane-1,3-dione présentent un effet antiradicalaire maximal de plus de 95 % dans le modèle utilisant le DPPH.

### Toxicité

Par ailleurs, les composés de formule (A) et leurs sels éventuels sont très peu toxiques. Par exemple, après administration unique per os chez la souris, aucune mortalité n'est observée à la dose de 1 g/kg avec la 2-phénylhydrazonoindane-1,3-dione. Les seuls effets observés sont dans certains cas des diarrhées colorées et des urines colorées, ces dernières étant le témoin d'une résorption du produit.

Ce qui précède montre que les composés de formule (A) et leurs sels éventuels peuvent être utilisés en thérapeutique humaine et animale. Ils sont en particulier indiqués dans l'insuffisance veineuse fonctionnelle, organique et les pathologies hémorroîdaires par leurs composantes vasculaires et antiinflammatoires, ainsi que dans les affections typiquement inflammatoires (ostéoarticulaires, dermatologiques ou cardiovasculaires) et dans les états de chocs constitués par une chute importante de la pression artérielle, en particulier dans les états de chocs septiques (endotoxiques).

L'insuffisance veineuse fonctionnelle se caractérise par une dilatation et une hyperdistensibilité des veines superficielles des membres inférieurs accompagnées par des symptômes fonctionnels : douleurs des membres inférieurs, oedèmes, paresthésies à type d'impatiences, de jambes sans repos. Ce type de pathologie peut évoluer vers l'insuffisance veineuse organique (varices, incontinence valvulaire profonde ...) voire vers la phlébothrombose et les lésions ulcéreuses.

Dans cette pathologie veineuse, une composante inflammatoire s'installe dans les premiers stades et se manifeste plus clairement dans les stades avancés.

La présente invention comprend donc l'utilisation des composés de formule (A) ci-dessus décrits et de leurs sels éventuels, comme substances actives pour la préparation de médicaments et compositions pharmaceutiques, à usage humain ou vétérinaire, comprenant au moins un desdits composés et sels en association avec un support ou diluant physiologiquement acceptable.

La forme de ces médicaments et compositions pharmaceutiques dépendra bien évidemment de la voie d'administration souhaitée notamment orale, parentérale et rectale et ils peuvent être formulés selon les techniques classiques avec mise en oeuvre des supports et véhicules usuels.

Ainsi, dans le cas d'une administration par voie orale, ils peuvent se présenter sous la forme de comprimés, tablettes, gélules, solutions, sirops et suspensions.

Les comprimés, tablettes et gélules contiennent la substance active conjointement avec un diluant (par exemple lactose, dextrose, sucrose, mannitol, sorbitol ou cellulose), un lubrifiant (par exemple silice, talc ou stéarate comme le stéarate de magnésium), un liant (par exemple amidon, méthylcellulose ou gomme arabique), un agent de désintégration (alginate par exemple) et ils sont fabriqués par des techniques connues par exemple de mélange, de granulation de pastillage. d'enrobage, etc...

Les sirops peuvent contenir, à titre de support, glycérol, mannitol et/ou sorbitol. Les solutions et suspensions peuvent comprendre de l'eau et un support tel qu'une gomme naturelle, de la gélose, de l'alginate de sodium ou de l'alcool polyvinylique.

Pour l'administration par voie parentérale, les médicaments et compositions peuvent prendre la forme de solutions, d'émulsions ou de suspensions comprenant la substance active et un support approprié tel que l'eau stérile ou des solutions salines isotoniques aqueuses stériles.

Pour l'administration par voie rectale, ils peuvent prendre la forme de suppositoires comprenant la substance active et un support approprié tel que le beurre de cacao ou du polyéthylène glycol.

La dose thérapeutique des substances actives pourra atteindre 1000 mg/jour suivant la voie d'administration, l'âge, le poids et l'état du sujet à traiter et la puissance thérapeutique de la substance active mise en oeuvre.

## Revendications

1. Composés répondant à la formule : 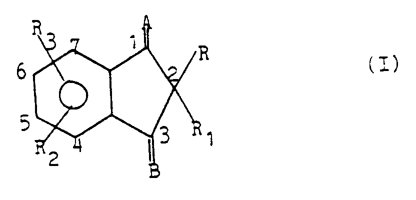 dans laquelle R₂ et R₃ représentent indépendamment l'un de l'autre H, alkoxy en C₁-C₄ ou OH ; et le couple (A, B) prend la valeur :
- (oxygène, oxygène), auquel cas l'un parmi R et R₁ représente OH, halogène, (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène et l'autre représente un groupe choisi parmi les suivants :
. NHCOR₄ où R₄ = phényle ; alkyle en C₁-C₄ ; ou alkyle en C₁-C₄ substitué par un halogène,
. NHNHCOR₅ où R₅ = alkoxy en C₁-C₄ ; phényle, phényle substitué par un ou deux groupes choisis parmi les suivants : amino, alkoxy en C₁-C₄, alkyle en C₁-C₄, OH : thiényle ; furyle ; pyridyle : indolyl-2-méthyle ; indolyl-3-méthyle ; ou 5-phényl-2-(N-pyrrolyl)thiényle,
. NHNHCOCONH₂,
à l'exclusion des dérivés pour lesquels l'un parmi R et R₁ représente OH ou halogène et l'autre représente NHCOR₄ où R₄ = phényle ou alkyle en C₁-C₄ ; des dérives pour lesquels l'un parmi R et R₁ représente (alkyl en C₁-C₄)NH et l'autre représente NHCOR₄ où R₄ = alkyle en C₁-C₄ ; et des dérivés pour lesquels l'un parmi R et R₁ représente OH et l'autre représente NHNHCOR₅ où R₅ = pyridyle ou phényle substitué par un groupe alkoxy en C₁-C₄,
R et R₁ pouvant par ailleurs former ensemble un groupe :
. =NNHCOR'₅ où R'₅ = R₅, trifluoroacétylaminophényle, acétylaminophényle, pyrazinyle ou pyrrolyle,
. =NNHCOCONH₂,
. =NNHR₆ où R₆ = phényle ; phényle substitué par un ou deux groupes choisis parmi alkyle en C₁-C₄, halogène et alkoxy en C₁-C₄ ; méthylsulfonylphényle ; et N-méthylméthanesulfonamidophényle,
. =N-R₇ où R₇ = phényle ou phényle substitué par un groupe OH,
. =N-O-CO-R₈ où R₈ = phényle ou phényle substitué par un atome d'halogène, ou
. =C(CH₃)-NH-NH-CO-R₉ où R₉ = phényle, phénylamino, phényle substitué par un atome d'halogéne ou un groupe OH, ou phénylamino substitué par un atome d'halogéne ou un groupe OH,
à l'exception des dérivés pour lesquels R et R₁ forment ensemble un groupe =NNHCOR'₅ où R'₅ = pyridyle ou phényle substitué par alkoxy en C₁-C₄ et des dérivés pour lesquels R et R₁ forment ensemble un groupe =N-NHR₆ où R₆ = phényle ou phényle substitué par un atome d'halogène ou un groupe alkyle ou alkoxy en C₁-C₄ ; ou
- (NNHCXNHR₁₀, oxygène) où X représente oxygène ou soufre et R₁₀ = H, phényle ou phényle substitué par un ou deux groupes choisis parmi OH, CF₃, alkyle en C₁-C₄, alkoxy en C₁-C₄, halogène, méthylènedioxy, acétoxy et hydroxyéthyle, auquel cas R et R₁ forment ensemble un groupe =N-OH,
ainsi que les sels d'addition d'acide des composés salifiables parmi ceux de formule (I).

2. Composés selon la revendication 1, caractérisés en ce qu'ils répondent à la formule : 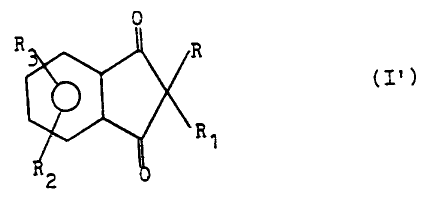 où R, R₁, R₂ et R₃ ont les mêmes significations que dans la formule (I) quand le couple (A, B) y prend la valeur (oxygène, oxygène).

3. Composés selon la revendication 1, caractérisés en ce qu'ils répondent à la formule : 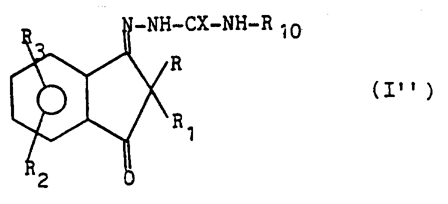 où R, R₁, R₂, R₃, X et R₁₀ ont les mêmes significations que dans la formule (I) quand le couple (A, B) y prend la signification (NNHCXNHR₁₀, oxygène).

4. Composés selon la revendication 1, caractérisés en ce qu'ils répondent à l'une des formules suivantes :
(a) 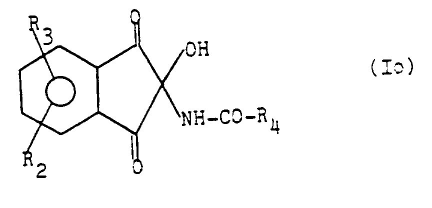 où R₄ = alkyle en C₁-C₄ substitué par un halogène.
(b) 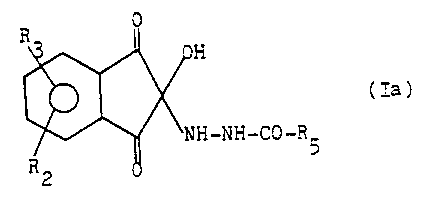 où R₅ = pyridyle et phényle substitué par un groupe alkoxy en C₁-C₄.
(c) 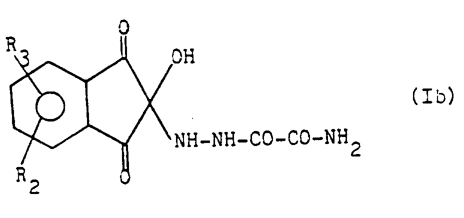
(d) 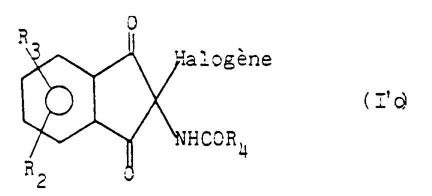 où R₄ = alkyle en C₁-C₄ substitué par un halogéne.
(e) 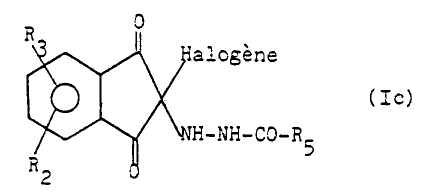
(f) 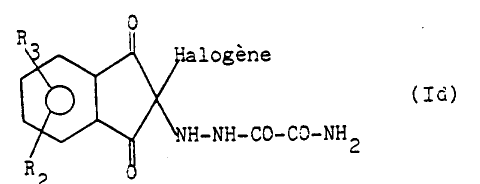
(g) 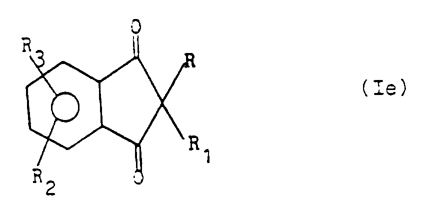 où l'un parmi R et R₁ représente NHCOR₄ et l'autre représente (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène, à l'exception des composés pour lesquels l'un parmi R et R₁ représente NHCOR₄ où R₄ = alkyle en C₁-C₄ et l'autre représente (alkyl en C₁-C₄)NH.
(h) 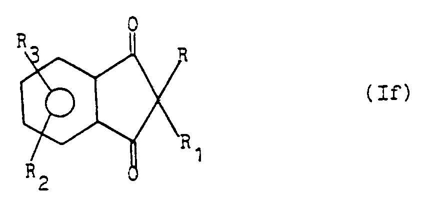 où l'un parmi R et R₁ représente NHNHCOR₅ et l'autre représente (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène.
(i) 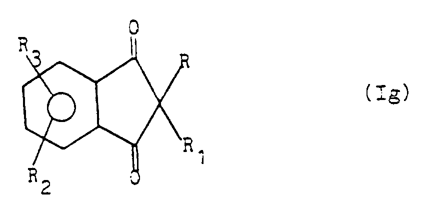 où l'un parmi R et R₁ représente NHNHCOCONH₂ et l'autre représente (alkyl en C₁-C₄)NH, morpholino(alkyl en C₁-C₄)NH, 1-(pyridiyl)-4-pipérazino ou 1-(phényl)-4-pipérazino dont le noyau phényle est éventuellement substitué par un halogène.
(j) 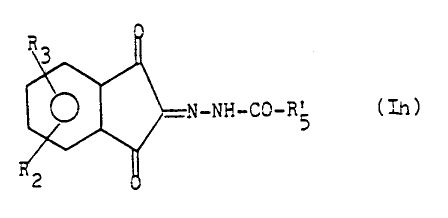 à l'exception de ceux pour lesquels R'₅ = phényle substitué par alkoxy en C₁-C₄ ; ou pyridyle.
(k) 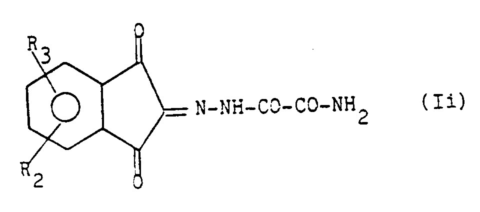
(l) 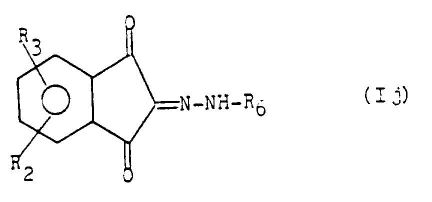 à l'exception des composés pour lesquels R₆ = phényle ou phényle substitué par un atome d'halogène ou un groupe alkyle ou alkoxy en C₁-C₄.
(m) 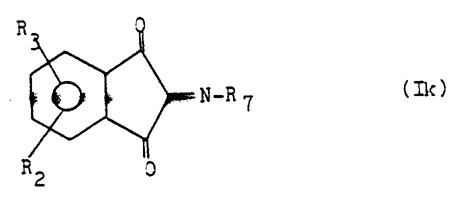
(n) 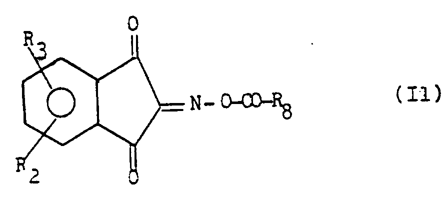
(o) 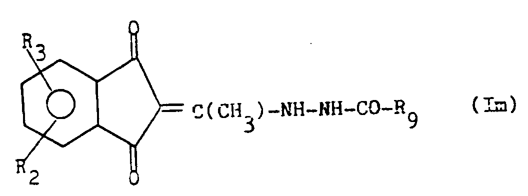
(p) 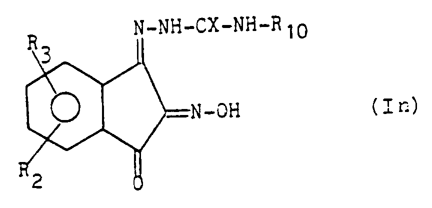 les symboles R₂, R₃, R₅, R'₅, R₆, R₇, R₈, R₉, R₁₀ et X ayant les mêmes significations que dans la formule (I).

5. Utilisation pour la fabrication d'un médicament, d'une part des composés de formule : 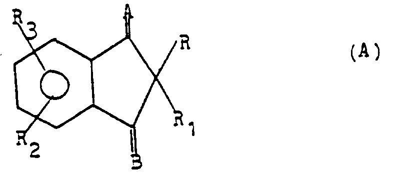 dans laquelle R₂ et R₃ représentent indépendamment l'un de l'autre H, alkoxy en C₁-C₄ ou OH ; et le couple (A, B) prend la valeur :
- (oxygène, oxygène), auquel cas l'un parmi R et R₁ représente OH, halogène, (alkyl en C₁-C₄)NH, N-morpholino(alkyl en C₁-C₄)NH, 1-(pyridyl)-4-pipérazino ou 1-(phényl)-4-pipérazino dont le noyau phényle est éventuellement substitué par un halogène et l'autre représente un groupe choisi parmi les suivants :
. NHCOR⁴ où R⁴ = phényle ; alkyle en C₁-C₄ ; ou alkyle en C₁-C₄ substitué par un halogène,
. NHNHCOR₅ où R₅ = alkoxy en C₁-C₄ ; phényle, phényle substitué par un ou deux groupes choisis parmi les suivants : amino, alkoxy en C₁-C₄, alkyle en C₁-C₄, OH ; thiényle ; furyle ; pyridyle ; indolyl-2_méthyle ; indolyl-3-méthyle ; ou 5-phényl-2-(N-pyrrolyl)thiényle,
. NHNHCOCONH₂,
R et R₁ pouvant par ailleurs former ensemble un groupe :
. =NNHCOR'₅ où R'₅ = R₅, trifluoroacétylaminophényle, acétylaminophényle, pyrazinyle ou pyrrolyle,
. =NNHCOCONH₂,
. =NNHR₆ où R₆ = phényle ; phényle substitué par un ou deux groupes choisis parmi alkyle en C₁-C₄, halogène et alkoxy en C₁-C₄ ; méthylsulfonylphényle ; et N-méthylméthanesulfonamidophényle,
. =N-R₇ où R₇ = phényle ou phényle substitué par un groupe OH,
. =N-O-CO-R₈ où R₈ = phényle ou phényle substitué par un atome d'halogène, ou
. =C(CH₃)-NH-NH-CO-R₉ où R₉ = phényle, phénylamino, phényle substitué par un atome d'halogène ou un groupe OH, ou phénylamino substitué par un atome d'halogène ou un groupe OH, ou
- (NNHCXNHR₁₀, oxygène) où X représente oxygène ou soufre et R₁₀ = H, phényle ou phényle substitué par un ou deux groupes choisis parmi OH, CF₃, alkyle en C₁-C₄, alkoxy en C₁-C₄, halogène, méthylènedioxy, acétoxy et hydroxyéthyle, auquel cas R et R₁ forment ensemble un groupe =N-OH,
à l'exclusion des composés pour lesquels R et R₁ forment ensemble un groupe =N-NH-CO-R'₅ où R'₅ = pyridyle ; et d'autre part des sels d'addition d'acide minéral ou organique, pharmaceutiquement acceptables des composés salifiables parmi ceux de formule (A) ci-dessus.

6. Composition pharmaceutique, caractérisée en ce qu'elle comprend un support ou diluant physiologiquement acceptable et au moins un composé ou sel pharmaceutiquement acceptable de ce composé, tel que défini à la revendication 5.

7. Procédé de préparation des composés de formule (I) selon la revendication 1, pour lesquels le couple (A, B) = (oxygène, oxygène), l'un parmi R et R₁ représente OH ou halogène et l'autre représente NHCOR₄, NHNHCOR₅ ou NHNHCOCONH₂, caractérisé en ce qu'il comprend la condensation des composés de formule :
H₂N-CO-R₄,
H₂N-NH-CO-R₅, ou
H₂N-NH-CO-CO-NH₂
sur l'indane-1,2,3-trione monohydrate de formule : 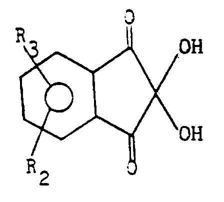 R₂ ,R₃, R₄ et R₅ ayant la même signification que dans la revendication 1, éventuellement suivie de la réaction des composés ainsi obtenus, avec un agent halogénant.

8. Procédé de préparation des composés de formule (I) selon la revendication 1, pour lesquels le couple (A, B) = (oxygène, oxygène), l'un parmi R et R₁ représente (alkyle en C₁-C₄), N-morpholino (alkyl en C₁-C₄)NH, 1-(pyridyl)-4-pipérazino ou 1-(phényl)-4-pipérazino dont le noyau phényle est éventuellement substitué par un halogène et l'autre représente NHCOR₄, NHNHCOR₅ ou NHNHCOCONH₂, caractérisé en ce qu'il comprend la condensation de (alkyl en C₁-C₄)NH₂, N-morpholino (alkyl en C₁-C₄)NH₂, 1-(pyridyl)-4-pipérazine ou 1-(phényl)-4-pipérazine dont le noyau phényle est éventuellement substitué par un halogène, respectivement sur les composés de formule : 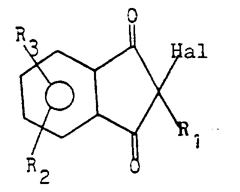 où R₂ et R₃ ont la même signification que dans la revendication 1, Hal = halogène et R₁ = NHCOR₄, NHNHCOR₅ ou NHNHCOCONH₂.

9. Procédé de préparation des composés de formule (I), selon la revendication 1, pour lesquels le couple (A, B) = (oxygène, oxygène) et R et R₁ forment ensemble un groupe =N-NH-CO-R'₅ ou =N-NH-CO-CO-NH₂, caractérisé en ce qu'il comprend le chauffage des composés de formule (I) selon la revendication 1 pour lesquels le couple (A, B) = (oxygène, oxygène) et l'un parmi R et R₁ représente OH et l'autre représente NH-NH-CO-R₅ ou NH-NH-CO-CO-NH₂, dans l'éthanol en présence d'HCl concentré ou dans l'acétonitrile, et éventuellement l'action d'un mélange d'acide trifluoroacétique et d'anhydride trifluoroacétique ou d'un mélange d'acide acétique et d'anhydride acétique sur les composés ainsi obtenus.

10. Procédé de préparation des composés de formule (I), selon la revendication 1, pour lesquels le couple (A, B) = (oxygène, oxygène) et R et R₁ forment ensemble un groupe =N-NH-CO-R'₅, =N-NH-R₆ ou =NH-R₇, caractérisé en ce qu'il comprend la condensation à chaud de H₂N-NH-CO-R'₅, du chlorhydrate de H₂N-NH-R₆ ou du chlorhydrate de H₂N-R₇ sur l'indane-1,2,3-trione monohydrate de formule : 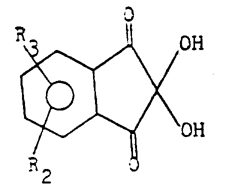 R₂, R₃, R'₅, R₆ et R₇ ayant la même signification que dans la revendication 1.

11. Procédé de préparation des compositions de formule (I), selon la revendication 1, pour lesquels le couple (A, B) = (oxygène, oxygène) et R et R₁ forment ensemble un groupe =N-O-CO-R₈, caractérisé en ce qu'il comprend la condensation du chloroformate d'isobutyle respectivement sur les composés de formule :
R₈ - COOH,
suivie de la condensation de l'anhydride mixte résultant, sur le composé de formule : 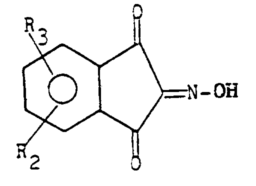 R₂, R₃ et R₈ ayant les mêmes sigifications que dans la formule (I).

12. Procédé de préparation des composés de formule (I), selon la revendication 1, pour lesquels le couple (A, B) = (oxygène, oxygène) et R et R₁ forment ensemble un groupe =C(CH₃)-NH-NH-CO-R₉, caractérisé en ce qu'il comprend la condensation des composés de formule :
R₉ - CO - NH - NH₂
respectivement sur les composés de formule : 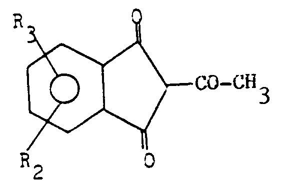 R₂, R₃ et R₉ ayant les mêmes significations que dans la formule (I).

13. Procédé de préparation des composés de formule (I), selon la revendication 1, pour lesquels le couple (A, B) = (N-NH-CX-NH-R₁₀, oxygène) et R et R₁ forment ensemble un groupe =N-OH, caractérisé en ce qu'il comprend la condensation du composé de formule :
H₂N - NH - CX - NH - R₁₀
sous forme de chlorhydrate, respectivement sur les composés de formule: 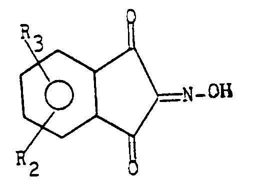 R₂, R₃, X et R₁₀ ayant les mêmes significations que des la formule (I).

## Claims

1. Compounds having the formula: 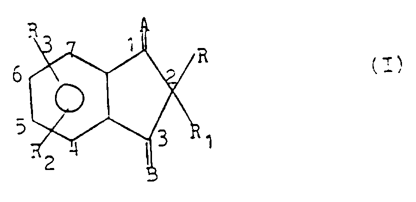 in which R₂ and R₃ independently denote H, C₁-C₄ alkoxy or OH; and the pair (A, B) denotes :
- (oxygen, oxygen), in which case one out of R and R₁ denotes OH, halogen, (C₁-C₄ alkyl) NH, N-morpholino(C₁-C₄ alkyl) NH, 1-(pyridyl)-4-piperazino or 1-(phenyl)-4-piperazino in which the phenyl ring is optionally substituted by a halogen, and the other denotes a group chosen from among the following:
. NHCOR₄ where R₄ = phenyl ; C₁-C₄ alkyl; or C₁-C₄ alkyl substituted by a halogen,
. NHNHCOR₅ where R₅ = C₁-C₄ alkoxy; phenyl, phenyl substituted by one or two groups chosen from among the following: amino, C₁-C₄ alkoxy, C₁-C₄ alkyl, OH; thienyl; furyl; pyridyl; indolyl-2-methyl; indolyl-3-methyl; or 5-phenyl-2-(N-pyrrolyl)thienyl,
. NHNHCOCONH₂,
excluding derivatives in which one out of R and R₁ represents OH or halogen and the other represents NHCOR₄ where R₄ = phenyl or C₁-C₄ alkyl; derivatives in which one out of R and R₁ denotes (C₁-C₄ alkyl)NH and the other denotes NHCOR₄ where R₄ = C₁-C₄ alkyl; and derivatives in which one out of R and R₁ denotes OH and the other denotes NHNHCOR₅ where R₅ = pyridyl or phenyl substituted by a C₁-C₄ alkoxy group, and R and R₁ may also together form a group, i.e: :
. =NNHCOR'₅ where R'₅ = R₅, trifluoroacetyl aminophenyl, acetyl aminophenyl, pyrazinyl or pyrrolyl,
. =NNHCOCONH₂,
. =NNHR₆ where R₆ = phenyl; phenyl substituted by one or two groups chosen from among C₁-C₄ alkyl, halogen and C₁-C₄ alkoxy; methyl sulphonyl phenyl; or N-methyl methane sulphonamidophenyl,
. =N-R₇ where R₇ = phenyl or phenyl substituted by an OH group,
. =N-O-CO-R₈ where R₈ = phenyl or phenyl substituted by a halogen atom,or
. =C(CH₃)-NH-NH-CO-R₉ where R₉ = phenyl, phenyl amino, phenyl substituted by a halogen atom or an OH group, or phenylamino substituted by a halogen atom or an OH group,
excepting derivatives in which R and R₁ together form an =NNHCOR'₅ group where R'₅ = pyridyl or phenyl substituted by C₁-C₄ alkoxy and derivatives in which R and R₁ together form an =N-NHR₆ group where R₆ = phenyl or phenyl substituted by a halogen atom or a C₁-C₄ alkyl or alkoxy group; or
- (NNHCXNHR₁₀, oxygen) where X denotes oxygen or sulphur and R₁₀ = H, phenyl or phenyl substituted by one or two groups chosen from among OH, CF₃, C₁-C₄ alkyl, C₁-C₄ alkoxy, halogen, methylene dioxy, acetoxy and hydroxyethyl, in which case R and R₁ together form an =N-OH group,
and acid addition salts of those formula (I) compounds which are salt-forming.

2. Compounds according to claim 1, characterised in that they have the formula: 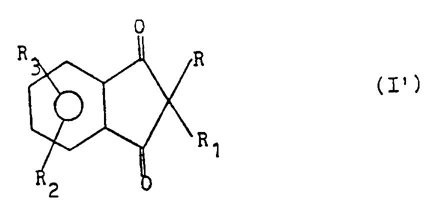 where R, R₁, R₂ and R₃ have the same meanings as in formula (I) when the pair (A, B) therein denotes (oxygen, oxygen).

3. Compounds according to claim 1, characterised in that they have the formula: 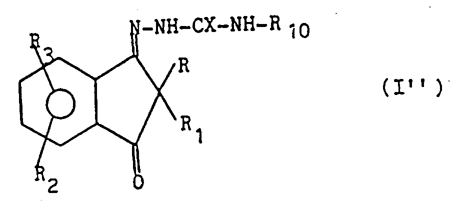 where, R, R₁, R₂, R₃, X and R₁₀ have the same meanings as in formula (I), when the pair (A, B) therein denotes (NNHCXNHR₁₀, oxygen).

4. Compounds according to claim 1, characterised in that they have one of the following formulae:
(a) 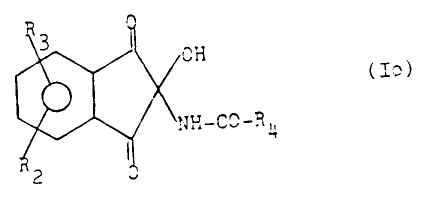 where R₄ = C₁-C₄ alkyl substituted by a halogen.
(b) 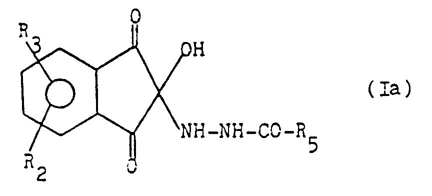 where R₅ ≠ pyridyl and phenyl substituted by a C₁-C₄ alkoxy group.
(c) 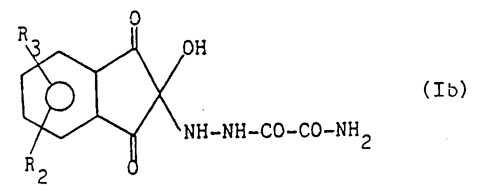
(d) 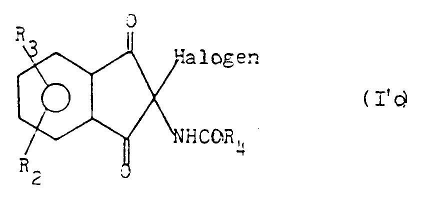 where R₄ = C₁-C₄ alkyl substituted by a halogen.
(e) 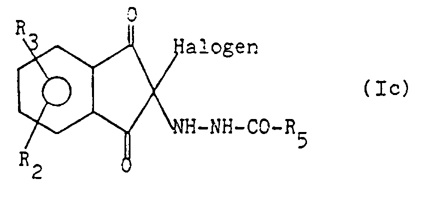
(f) 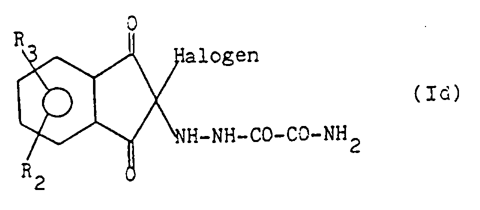
(g) 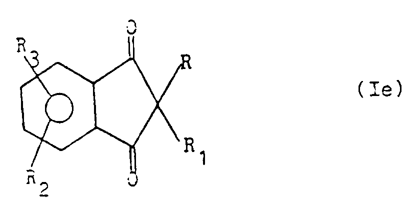 where one out of R₁ and R₂ denotes NHCOR₄ and the other denotes (C₁-C₄ alkyl) NH, N-morpholino (C₁-C₄ alkyl) NH, 1-(pyridyl)-4-piperazino or 1-(phenyl)-4-piperazino in which the phenyl ring is optionally substituted by a halogen, except for compounds in which one out of R and R₁ denotes NHCOR₄ where R₄ = C₁-C₄ alkyl and the other denotes (C₁-C₄ alkyl) NH.
(h) 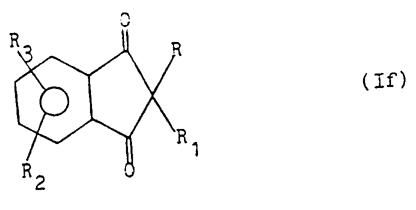 where one out of R and R₁ denotes NHNHCOR₅ and the other denotes (C₁-C₄ alkyl) NH, N-morpholino (C₁-C₄ alkyl) NH, 1-(pyridyl)-4-piperazino or 1-(phenyl)-4-piperazino in which the phenyl ring is optionally substituted by a halogen.
(i) 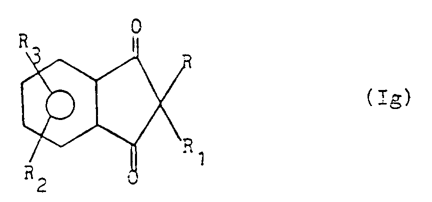 where one out of R and R₁ denotes NHNHCOCONH₂ and the other denotes (C₁-C₄ alkyl)NH , morpholino (C₁-C₄ alkyl) NH, 1-(pyridyl)-4-piperazino or 1-(phenyl)-4-piperazino in which the phenyl ring is optionally substituted by a halogen.
(j) 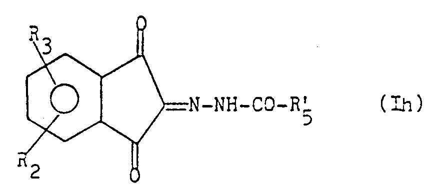 apart from those in which R'₅ = phenyl substituted by C₁-C₄ alkoxy; or pyridyl.
(k) 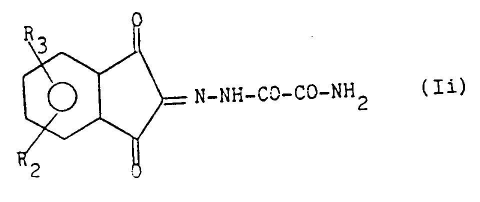
(l) 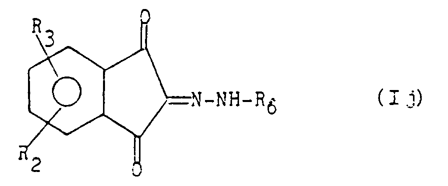 except for compounds in which R₆ = phenyl or phenyl substituted by a halogen atom or a C₁-C₄ alkyl or alkoxy group.
(m) 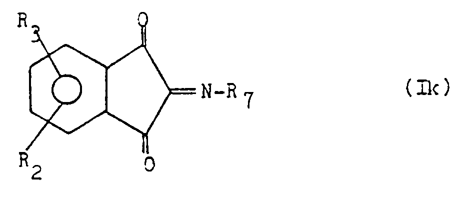
(n) 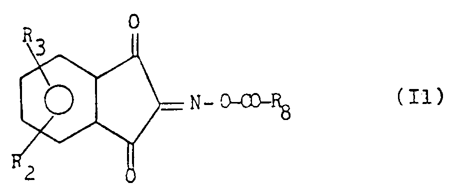
(o) 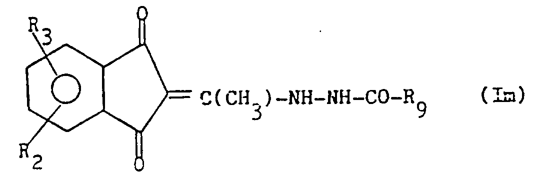
(p) 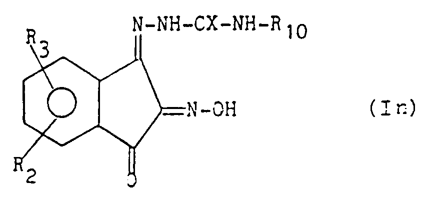 the symbols R₂, R₃, R₅, R'₅, R₆, R₇, R₈, R₉, R₁₀ and X having the same meanings as in formula (I).

5. Use, for manufacture of a drug, one the one hand of the compounds having the formula: 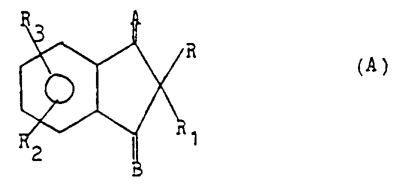 in which R₂ and R₃ independently denote H, C₁-C₄ alkoxy or OH; and the pair (A, B) denotes :
- (oxygen, oxygen), in which case one out of R and R₁ denotes OH, halogen, (C₁-C₄ alkyl) NH, N-morpholino(C₁-C₄ alkyl) NH, 1-(pyridyl)-4-piperazino or 1-(phenyl)-4-piperazino in which the phenyl ring is optionally substituted by a halogen, and the other denotes a group chosen from among the following:
. NHCOR₄ where R₄ = phenyl; C₁-C₄ alkyl; or C₁-C₄ alkyl substituted by a halogen,
. NHNHCOR₅ where R₅ = C₁-C₄ alkoxy; phenyl, phenyl substituted by one or two groups chosen from among the following: amino, C₁-C₄ alkoxy, C₁-C₄ alkyl, OH; thienyl; furyl; pyridyl; indolyl-2-methyl; indolyl-3-methyl; or 5-phenyl-2-(N-pyrrolyl)thienyl,
. NHNHCOCONH₂, and
R and R₁ may also together form a group, i.e.:
. =NNHCOR'₅ where R'₅ = R₅, trifluoroacetyl aminophenyl, acetyl aminophenyl, pyrazinyl or pyrrolyl,
. =NNHCOCONH₂,
. =NNHR₆ where R₆ = phenyl; phenyl substituted by one or two groups chosen from among C₁-C₄ alkyl, halogen and C₁-C₄ alkoxy; methyl sulphonyl phenyl; or N-methyl methane sulphonamidophenyl,
. =N-R₇ where R₇ = phenyl or phenyl substituted by an OH group,
. =N-O-CO-R₈ where R₈ = phenyl or phenyl substituted by a halogen atom or
. =C(CH₃)-NH-NH-CO-R₉ where R₉ = phenyl, phenyl amino, phenyl substituted by a halogen atom or an OH group, or phenylamino substituted by a halogen atom or an OH group, or
- (NNHCXNHR₁₀, oxygen) where X denotes oxygen or sulphur and R₁₀ = H, phenyl or phenyl substituted by one or two groups chosen from among OH, CF₃, C₁-C₄ alkyl, C₁-C₄ alkoxy, halogen, methylene dioxy, acetoxy and hydroxyethyl, in which case R and R₁ together form an =N-OH group,
except for compounds in which R and R₁ together form an =N-NH-CO-R'₅ group where R'₅ = pyridyl;
and on the other hand of pharmaceutically acceptable mineral or organic acid addition salts of the salt-forming compounds among those having the formula (A) hereinbefore.

6. A pharmaceutical composition characterised in that it comprises a physiologically acceptable excipient or diluent and at least one compound or pharmaceutically acceptable salt of this compound, as defined in claim 5.

7. A method of preparing formula (I) compounds according to claim 1,in which the pair (A, B) = (oxygen, oxygen), one out of R and R₁ denotes OH or halogen and the other denotes NHCOR₄, NHNHCOR₅ or NHNHCOCONH₂, characterised in that it comprises condensation of compounds having the formula:
H₂N-CO-R₄,
H₂N-NH-CO-R₅, or
H₂N-NH-CO-CO-NH₂
on indan-1,2,3-trione monohydrate having the formula: 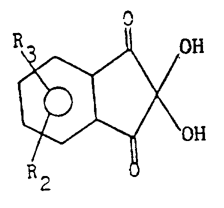 R₂, R₃, R₄ and R₅ having the same meanings as in claim 1, optionally followed by reaction of the resulting compounds with a halogenating agent.

8. A method of preparing formula (I) compounds according to claim 1, in which the pair (A, B) = (oxygen, oxygen), one out of R and R₁ denotes (C₁-C₄ alkyl)NH, N-morpholino (C₁-C₄ alkyl) NH, 1-(pyridyl)-4-piperazino or 1-(phenyl)-4-piperazino in which the phenyl ring is optionally substituted by a halogen and the other denotes NHCOR₄, NHNHCOR₅ or NHNHCOCONH₂, characterised in that it comprises condensation of (C₁-C₄ alkyl) NH₂, N-morpholino (C₁-C₄ alkyl) NH₂, 1-(pyridyl)-4-piperazine or 1-(phenyl)-4-piperazine in which the phenyl ring is optionally substituted by a halogen, respectively, on compounds having the formula: 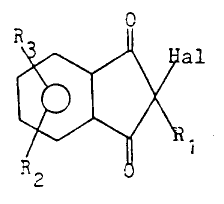 where R₂ and R₃ have the same meanings as in claim 1, Hal = halogen and R₁ = NHCOR₄, NHNHCOR₅ or NHNHCOCONH₂.

9. A method of preparing formula (I) compounds according to claim 1, in which the pair (A, B) = (oxygen, oxygen) and R and R₁ together form an =N-NH-CO-R'₅ or =N-NH-CO-CO-NH₂ group, characterised in that it comprises heating formula (I) compounds according to claim 1, in which the pair (A, B) = (oxygen, oxygen) and one out of R and R₁ denotes OH and the other denotes NH-NH-CO-R₅ or NH-NH-CO-CO-NH₂, in ethanol in the presence of concentrated HCl or in acetonitrile, optionally with action of a mixture of trifluoroacetic acid and trifluoroacetic anhydride or a mixture of acetic acid and acetic anhydride on the resulting compounds.

10. A method of preparing formula (I) compounds according to claim 1, in which the pair (A, B) = (oxygen, oxygen) and R and R₁ together form an =N-NH-CO-R'₅, =N-NH-R₆ or =NH=R₇ group, characterised in that it comprises hot condensation of H₂N-NH-CO-R'₅_{,} H₂N-NH-R₆ hydrochloride or H₂N-R₇ hydrochloride on indan-1,2,3-trione monohydrate having the formula: 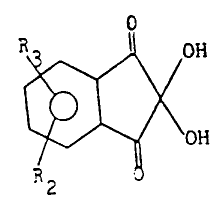 R₂, R₃, R'₅, R₆ and R₇ having the same meanings as in claim 1.

11. A method of preparing formula (I) compounds according to claim 1, in which the pair (A, B) = (oxygen, oxygen) and R and R₁ together form an =N-O-CO-R₈ group, characterised in that it comprises condensation of isobutyl chloroformate on the respective compounds having the formula:
R₈ - COOH
followed by condensation of the resulting mixed anhydride on the compound having the formula: 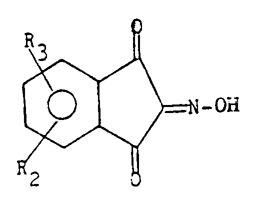 R₂, R₃ and R₈ having the same meanings as in formula (I).

12. A method of preparing formula (I) compounds according to claim 1, in which the pair (A, B) = (oxygen, oxygen) and R and R₁ together form an =C(CH₃)-NH-NH-CO-R₉ group, characterised in that it comprises condensation of compounds having the formula:
R₉ - CO - NH - NH₂
on the respective compounds having the formula: 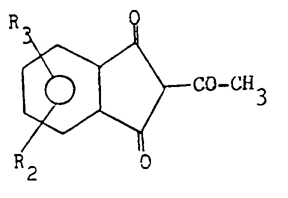 R₂, R₃ and R₉ having the same meanings as in formula (I).

13. A method of preparing formula (I) compounds according to claim 1, in which the pair (A, B) = (N-NH-CX-NH-R₁₀, oxygen) and R and R₁ together form an =N-OH group, characterised in that it comprises condensation of the compound having the formula:
H₂N - NH - CX - NH - R₁₀
in hydrochloride form, on the respective compounds having the formula: 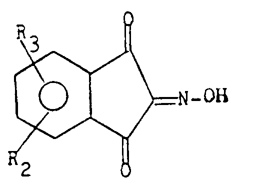 R₂, R₃, X and R₁₀ having the same meanings as in formula (I).

## Patentansprüche

1. Verbindungen, entsprechend der Formel: 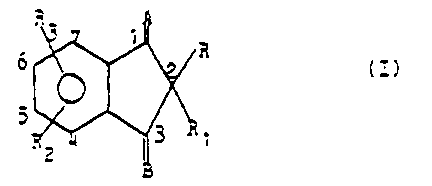 in welcher R₂ und R₃ unabhängig voneinander H, C₁-C₄-Alkoxy oder OH darstellen; und das Paar (A, B) den Wert annimmt:
- (Sauerstoff, Sauerstoff), wobei in diesem Fall eines von R und R₁ OH, Halogen, (C₁-C₄-Alkyl)NH, N-Morpholino(C₁-C₄-alkyl)NH, 1-(Pyridyl)-4-piperazino oder 1-(Phenyl)-4-piperazino, dessen Phenylkern gegebenenfalls mit einem Halogen substituiert ist, darstellt und das andere eine Gruppe darstellt, die unter den folgenden ausgewählt wird:
. NHCOR₄, wobei R₄ = Phenyl; C₁-C₄-Alkyl; oder C₁-C₄-Alkyl, das mit einem Halogen substituiert ist,
. NHNHCOR₅, wobei R₅ = C₁-C₄-Alkoxy; Phenyl, Phenyl, das mit einer oder zwei Gruppen substituiert ist, die unter den folgenden ausgewählt werden: Amino, C₁-C₄-Alkoxy, C₁-C₄-Alkyl, OH; Thienyl; Furyl; Pyridyl; Indolyl-2-methyl; Indolyl-3-methyl; oder 5-Phenyl-2-(N-Pyrrolyl)thienyl,
. NHNHCOCONH₂_{,}
mit Ausnahme der Derivate, für welche eines von R und R₁ OH oder Halogen darstellt und das andere NHCOR₄ darstellt, wobei R₄ = Phenyl oder C₁-C₄-Alkyl; der Derivate, für welche eines von R und R₁ (C₁-C₄-Alkyl)NH darstellt und das andere NHCOR₄ darstellt, wobei R₄ = C₁-C₄-Alkyl; und der Derivate, für welche eines von R und R₁ OH darstellt und das andere NHNHCOR₅ darstellt, wobei R₅ = Pyridyl oder Phenyl, das mit einer C₁-C₄-Alkoxy-Gruppe substituiert ist,
R und R₁ können außerdem zusammen eine Gruppe bilden:
. =NNHCOR'₅, wobei R'₅ = R₅, Trifluoracetylaminophenyl, Acetylaminophenyl, Pyrazinyl oder Pyrrolyl,
. =NNHCOCONH₂,
. =NNHR₆, wobei R₆ = Phenyl; Phenyl, das mit einer oder zwei Gruppen substituiert ist, die ausgewählt werden unter einem C₁-C₄-Alkyl, Halogen und C₁-C₄-Alkoxy; Methylsulfonylphenyl; und N-Methylmethansulfonamidophenyl,
. =N-R₇, wobei R₇ = Phenyl oder ein mit einer OH-Gruppe substituiertes Phenyl,
. =N-O-CO-R₈, wobei R₈ - Phenyl oder ein mit einem Halogenatom substituiertes Phenyl, oder
. =C(CH₃)-NH-NH-CO-R₉, wobei R₉ = Phenyl, Phenylamino, Phenyl, das mit einem Halogenatom oder einer OH-Gruppe substituiert ist, oder Phenylamino, das mit einem Halogenatom oder einer OH-Gruppe substituiert ist,
mit Ausnahme Von Derivaten, für welche R und R₁ zusammen eine Gruppe =NNHCOR'₅ bilden, wobei R'₅ = Pyridyl oder Phenyl, das mit C₁-C₄-Alkoxy substituiert ist, und von Derivaten, für welche R und R₁ zusammen eine Gruppe =N-NHR₆ bilden, wobei R₆ - Phenyl oder Phenyl, das mit einem Halogenatom oder einer C₁-C₄-Alkoxy- oder Alkyl-Gruppe substituiert ist; oder
- (NNHCXNHR₁₀, Sauerstoff), wobei X Sauerstoff oder Schwefel darstellt und R₁₀ = H, Phenyl oder Phenyl, das mit einer oder zwei Gruppen substituiert ist, ausgewählt unter OH, CF₃, C₁-C₄-Alkyl, C₁-C₄-Alkoxy, Halogen, Methylendioxy, Acetoxy und Hydroxyethyl, wobei in diesem Fall R und R₁ zusammen eine Gruppe =N-OH bilden,
sowie die Säuresalze von salzbildenden Verbindungen unter denen der Formel (I).

2. Verbindungen gemäß Anspruch 1, **dadurch gekennzeichnet**, daß sie der Formel: 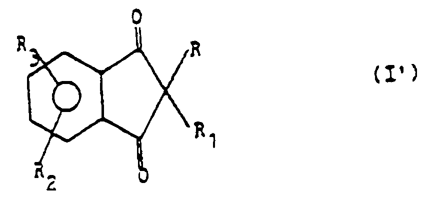 entsprechen, wobei R, R₁, R₂ und R₃ dieselben Bedeutungen haben wie in Formel (I), wenn das Paar (A, B) dort den Wert (Sauerstoff, Sauerstoff) annimmt.

3. Verbindungen gemäß Anspruch 1, **dadurch gekennzeichnet**, daß sie der Formel: 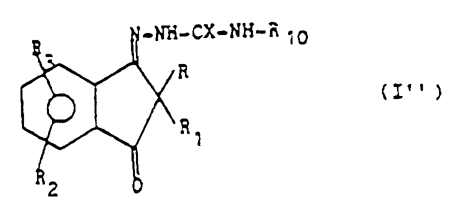 entsprechen, wobei R, R₁, R₂, R₃, X und R₁₀ dieselben Bedeutungen haben wie in Formel (I), wenn das Paar (A, B) dort die Bedeutung (NNHCXNHR₁₀, Sauerstoff) annimmt

4. Verbindungen gemäß Anspruch 1, **dadurch gekennzeichnet**, daß sie einer der folgenden Formeln entsprechen:
(a) 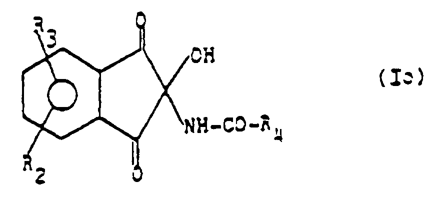 wobei R₄ = C₁-C₄-Alkyl, das durch ein Halogen substituiert ist.
(b) 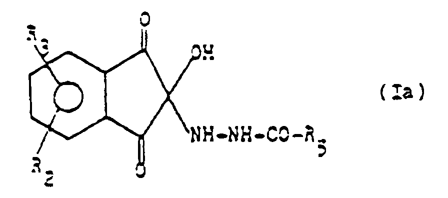 wobei R₅ = Pyridyl und Phenyl, das durch eine C₁-C₄-Alkoxy-Gruppe substituiert ist.
(c) 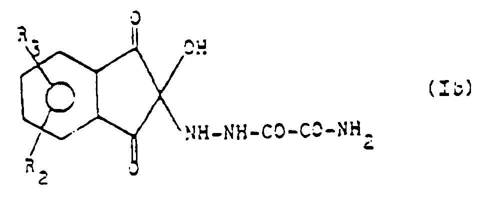
(d) 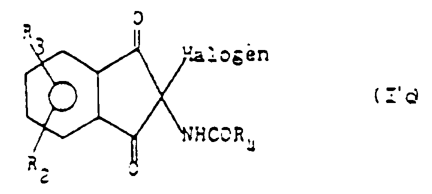 wobei R₄ = C₁-C₄-Alkyl, das durch ein Halogen substituiert ist.
(e) 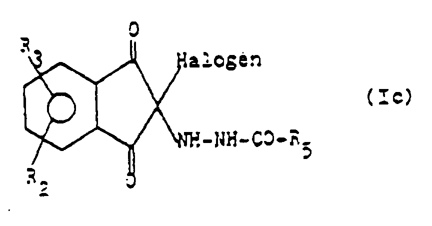
(f) 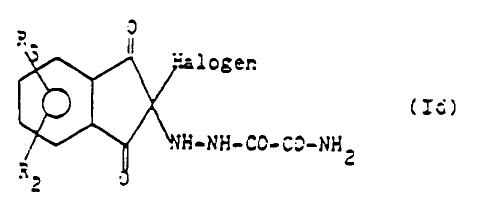
(g) 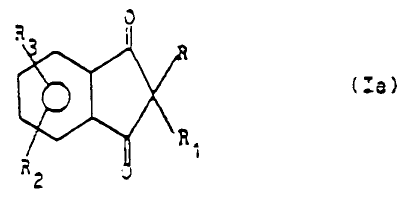 wobei eines von R und R₁ NHCOR₄ darstellt und das andere (C₁-C₄-Alkyl)NH, N-Morpholino(C₁-C₄-alkyl)NH, 1-(Pyridyl)-4-piperazino oder 1-(Phenyl)-4-piperazino darstellt, dessen Phenylkern gegebenenfalls mit einem Halogen substituiert ist, mit Ausnahme von Verbindungen, für welche eines von R und R₁ NHCOR₄ darstellt, wobei R₄ = C₁-C₄-Alkyl, und das andere (C₁-C₄-Alkyl)NH darstellt.
(h) 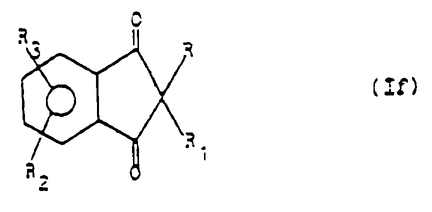 wobei eines von R und R₁ NHNHCOR₅ darstellt und das andere (C₁-C₄-Alkyl)NH, N-Morpholino(C₁-C₄-alkyl)NH, 1-(Pyridyl)-4-piperazino oder 1-(Phenyl)-4-piperazino darstellt, dessen Phenylkern gegebenenfalls mit einem Halogen substituiert ist.
(i) 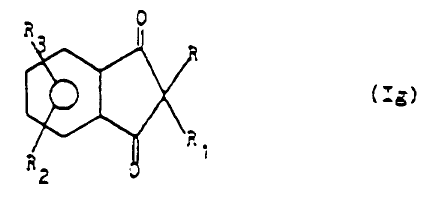 wobei eines von R und R₁ NHNHCOCONH₂ darstellt und das andere (C₁-C₄-Alkyl)NH, Morpholino(C₁-C₄-alkyl)NH, 1-(Pyridyl)-4-piperazino oder 1-(Phenyl)-4-piperazino darstellt, dessen Phenylkern gegebenenfalls mit einem Halogen substituiert ist.
(j) 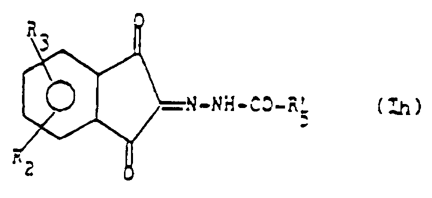 mit Ausnahme derjenigen, für welche R'₅ = ein mit einem C₁-C₄-Alkoxy substituiertes Phenyl; oder Pyridyl.
(k) 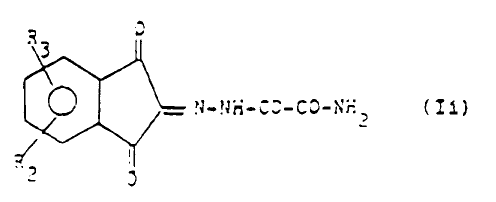
(l) 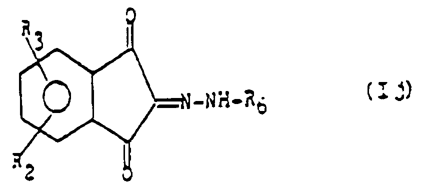 mit Ausnahme von Verbindungen, für welche R₆ = Phenyl, oder Phenyl, das durch ein Halogenatom oder eine C₁-C₄-Alkoxy- oder Alkyl-Gruppe substituiert ist.
(m) 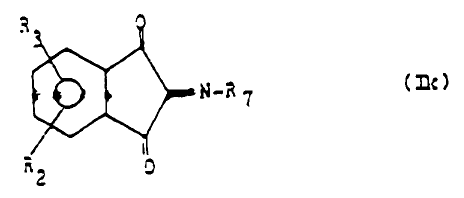
(n) 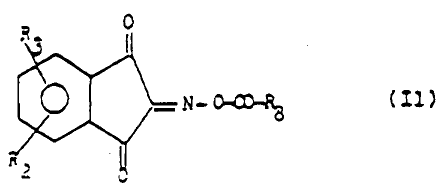
(o) 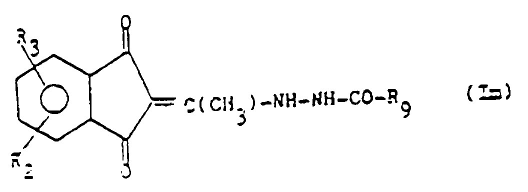
(p) 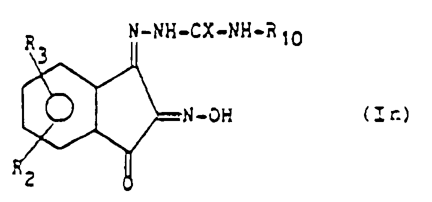 wobei die Symbole R₂, R₃, R₅, R'₅, R₆, R₇, R₈, R₉, R₁₀ und X dieselben Bedeutungen haben wie in Formel (I).

5. Verwendung für die Herstellung eines Medikamentes, einerseits der Verbindungen der Formel: 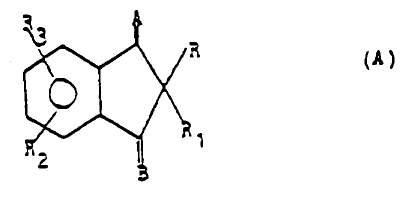 in welcher R₂ und R₃ unabhängig voneinander H, C₁-C₄-Alkoxy oder OH darstellen; und das Paar (A, B) den Wert annimmt:
- (Sauerstoff, Sauerstoff), wobei in diesem Fall eines von R und R₁ OH, Halogen, (C₁-C₄-Alkyl)NH, N-Morpholino(C₁-C₄-alkyl)NH, 1-(Pyridyl)-4-piperazino oder 1-(Phenyl)-4-piperazino darstellt, dessen Phenylkern gegebenenfalls mit einem Halogen substituiert ist, und das andere eine Gruppe darstellt, die unter den folgenden ausgewählt wird:
. NHCOR₄, wobei R₄ = Phenyl; C₁-C₄-Alkyl; oder C₁-C₄-Alkyl, das mit einem Halogen substituiert ist,
. NHNHCOR₅, wobei R₅ = C₁-C₄-Alkoxy; Phenyl, Phenyl, das mit einer oder zwei Gruppen substituiert ist, die unter den folgenden ausgewählt werden: Amino, C₁-C₄-Alkoxy, C₁-C₄-Alkyl, OH; Thienyl; Furyl; Pyridyl; Indolyl-2-methyl; Indolyl-3-methyl; oder 5-Phenyl-2-(N-Pyrrolyl)thienyl,
. NHNHCOCONH₂,
R und R₁ können außerdem zusammen eine Gruppe bilden:
. =NNHCOR'₅_{,} wobei R'₅ = R₅, Trifluoracetylaminophenyl, Acetylaminophenyl, Pyrazinyl oder Pyrrolyl,
. =NNHCOCONH₂,
. =NNHR₆, wobei R₆ = Phenyl; Phenyl, das mit einer oder zwei Gruppen substituiert ist, die ausgewählt werden unter C₁-C₄-Alkyl, Halogen und C₁-C₄-Alkoxy; Methylsulfonylphenyl; und N-Methylmethansulfonamidophenyl,
. =N-R₇, wobei R₇ = Phenyl oder ein mit einer OH-Gruppe substituiertes Phenyl,
. =N-O-CO-R₈, wobei R₈ = Phenyl oder ein mit einem Halogenatom substituiertes Phenyl, oder
. =C(CH₃)-NH-NH-CO-R₉, wobei R₉ = Phenyl, Phenylamino, Phenyl, das mit einem Halogenatom oder einer OH-Gruppe substituiert ist, oder Phenylamino, das mit einem Halogenatom oder einer OH-Gruppe substituiert ist,
- (NNHCXNHR₁₀, Sauerstoff), wobei X Sauerstoff oder Schwefel darstellt und R₁₀ = H, Phenyl oder Phenyl, das mit einer oder zwei Gruppen substituiert ist, ausgewählt unter OH, CF₃, C₁-C₄-Alkyl, C₁-C₄-Alkoxy, Halogen, Methylendioxy, Acetoxy und Hydroxyethyl, wobei in diesem Fall R und R₁ zusammen eine Gruppe =N-OH bilden,
mit Ausnahme von Derivaten, für welche R und R₁ zusammen eine Gruppe =N-NH-CO-R'₅ bilden, wobei R'₅ = Pyridyl; und andererseits der pharmazeutisch annehmbaren Salze von salzbildenden Verbindungen unter denen der oben angegebenen Formel (A) mit anorganischer oder organischer Säure.

6. Pharmazeutische Zusammensetzung, **dadurch gekennzeichnet**, daß sie einen Trägerstoff oder ein physiologisch annehmbares Verdünnungsmittel und mindestens eine Verbindung oder ein pharmazeutisch annehmbares Salz dieser Verbindung, wie in Anspruch 5 definiert, umfaßt.

7. Verfahren zur Herstellung von Verbindungen der Formel (I) gemäß Anspruch 1, für welche das Paar (A, B) = (Sauerstoff, Sauerstoff), wobei eines von R und R₁ OH oder Halogen darstellt, und das andere NHCOR₄, NHNHCOR₅ oder NHNHCOCONH₂ darstellt, **dadurch gekennzeichnet**, daß es die Kondensation von Verbindungen der Formel:
H₂N-CO-R₄,
H₂N-NH-CO-R₅, oder
H₂H-NH-CO-CO-NH₂
mit Indan-1,2,3-trion-monohydrat der Formel: 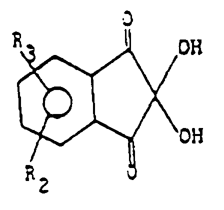 umfaßt, wobei R₂, R₃, R₄ und R₅ dieselbe Bedeutung haben wie in Anspruch 1, gegebenenfalls gefolgt von der Reaktion der somit erhaltenen Verbindungen mit einem Halogenierungsmittel.

8. Verfahren zur Herstellung von Verbindungen der Formel (I) gemäß Anspruch 1, für welche das Paar (A, B) = (Sauerstoff, Sauerstoff), wobei eines von R oder R₁ (C₁-C₄-Alkyl), N-Morpholino(C₁-C₄-alkyl)NH, 1-(Pyridyl)-4-piperazino oder 1-(Phenyl)-4-piperazino darstellt, dessen Phenylkern gegebenenfalls mit einem Halogen substituiert ist, und das andere NHCOR₄, NHNHCOR₅ oder NHNHCOCONH₂ darstellt, **dadurch gekennzeichnet**, daß es die Kondensation von (C₁-C₄-Alkyl)NH₂, N-Morpholino(C₁-C₄-alkyl)NH₂, 1-(Pyridyl)-4-piperazin oder 1-(Phenyl)-4-piperazin, dessen Phenylkern gegebenenfalls mit einem Halogen substituiert ist, jeweils mit den Verbindungen der Formel: 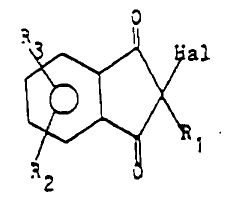 umfaßt, wobei R₂ und R₃ dieselbe Bedeutung haben wie in Anspruch 1, Hal = Halogen und R₁ = NHCOR₄, NHNHCOR₅ oder NHNHCOCONH₂.

9. Verfahren zur Herstellung von Verbindungen der Formel (I) gemäß Anspruch 1, für welche das Paar (A, B) = (Sauerstoff, Sauerstoff) und R und R₁ zusammen eine Gruppe =N-NH-CO-R'₅ oder =N-NH-CO-CO-NH₂ bilden, **dadurch gekennzeichnet**, daß es das Erhitzen der Verbindungen der Formel (I) gemäß Anspruch 1, für welche das Paar (A, B) = (Sauerstoff, Sauerstoff) und eines von R und R₁ OH darstellt und das andere NH-NH-CO-R₅ oder NH-NH-CO-CO-NH₂ darstellt, in Ethanol in Anwesenheit von konzentriertem HCl oder in Acetonitril, und gegebenenfalls das Einwirken einer Mischung aus Trifluoressigsäure und Trifluoressigsäureanhydrid oder einer Mischung aus Essigsäure und Essigsäureanhydrid auf die somit erhaltenen Verbindungen umfaßt.

10. Verfahren zur Herstellung von Verbindungen der Formel (I) gemäß Anspruch 1, für welche das Paar (A, B) = (Sauerstoff, Sauerstoff) und R und R₁ zusammen eine Gruppe =N-NH-CO-R'₅, =N-NH-R₆ oder =NH-R₇ bilden, **dadurch gekennzeichnet**, daß es die Kondensation von H₂N-NH-CO-R'₅, von H₂N-NH-R₆-Hydrochlorid oder H₂N-R₇-Hydrochlorid mit Indan-1,2,3-trion-monohydrat der Formel: 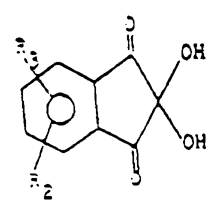 umfaßt, wobei R₂, R₃, R'₅, R₆ und R₇ dieselbe Bedeutung haben wie in Anspruch 1.

11. Verfahren zur Herstellung von Verbindungen der Formel (I), gemäß Anspruch 1, für welche das Paar (A, B) = (Sauerstoff, Sauerstoff) und R und R₁ zusammen eine Gruppe =N-O-CO-R₈ bilden, **dadurch gekennzeichnet**, daß es die Kondensation von Chlorameisensäureisobutylester jeweils mit den Verbindungen der Formel:
R₈-COOH,
gefolgt von der Kondensation des daraus resultierenden gemischten Anhydrids mit der Verbindung der Formel umfaßt: 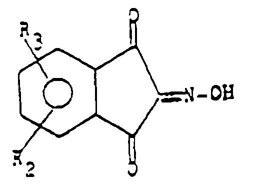 wobei R₂, R₃ und R₈ dieselben Bedeutungen haben wie in Formel (I).

12. Verfahren zur Herstellung von Verbindungen der Formel (I) gemäß Anspruch 1, für welche das Paar (A, B) = (Sauerstoff, Sauerstoff) und R und R₁ zusammen eine Gruppe =C(CH₃)-NH-NH-CO-R₉ bilden, **dadurch gekennzeichnet**, daß es die Kondensation der Verbindungen der Formel:
R₉-CO-NH-NH₂
jeweils mit den Verbindungen der Formel: 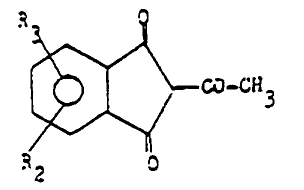 umfaßt, wobei R₂, R₃ und R₉ dieselben Bedeutungen haben wie in Formel (I).

13. Verfahren zur Herstellung von Verbindungen der Formel (I) gemäß Anspruch 1, für welche das Paar (A, B) = (N-NH-CX-NH-R₁₀, Sauerstoff) und R und R₁ zusammen eine Gruppe =N-OH bilden, **dadurch gekennzeichnet**, daß es die Kondensation der Verbindung der Formel:
H₂N-NH-CX-NH-R₁₀
in Form des Hydrochlorids jeweils mit den Verbindungen der Formel: 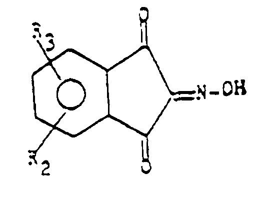 umfaßt, wobei R₂, R₃, X und R₁₀ dieselben Bedeutungen haben wie in Formel (I).
